# EUROPEAN PATENT APPLICATION

(11) **EP 4 825 967 A1**
(43) Date of publication of application: **21.10.2026**
(21) Application number: 24904419.9
(22) Date of filing: 13.12.2024
(51) Int. Cl.: C12N 15/113, A61K 48/00, A61K 31/7088, A61P 25/28, A61P 35/00

(54) **INHIBITION OF TARGET MRNA EXPRESSION USING CIRCULAR RNA**

(30) Priority: 14.12.2023 KR 20230181864
(71) Applicant: Korea Advanced Institute of Science and Technology, Daejeon 34141 (KR); Korea University Research and Business Foundation, Seoul 02841 (KR)
(72) Inventor: KIM, Yoon Ki, Daejeon 34141 (KR); HWANG, Hyun Jung, Daejeon 35211 (KR); BOO, Sung Ho, Seoul 04595 (KR); SHIN, Min-Kyung, Seoul 02463 (KR)
(74) Representative: Müller, Christian Stefan Gerd
(86) International application number: PCT/KR2024/096899
(87) International publication number: WO 2025/127854

(57) **Abstract**

The present invention relates to a new method for rapidly degrading target mRNA, wherein circular RNA containing an EJC at the 3' UTR of the target mRNA is artificially bound to the target mRNA to reduce the expression of the corresponding gene through selective degradation of the target mRNA.

## Description

### Technical Field

The present invention relates to the inhibition of target mRNA expression using circular RNA (Circular RNA).

### Background Art

Various biological functions occurring within eukaryotic cells are regulated by the central dogma, which involves the conversion of DNA into mRNA and then into proteins. In this process, the stability and quality of mRNA play a crucial role in determining the quantity and quality of the final protein produced, and are strictly regulated within the cell.

In addition to linear mRNA with well-known 5' caps and 3' poly(A) tails, circular RNA (circular RNA) exists in cells. Circular RNA is formed when back splicing occurs instead of the usual splicing, and unlike linear mRNA, it does not have a 5' cap or 3' poly(A) tail, giving it resistance to exoribonucleases and making it a more stable form.

On the other hand, nonsense-mediated mRNA decay (NMD) is a process that can be induced by proteins such as exon junction complex (EJC) factors and nonsense-mediated mRNA degradation factors like UPF1, UPF2, and PNRC2, which can induce the degradation of nonsense-mediated mRNA. EJC) factors and proteins such as UPF1, UPF2, and PNRC2, which can induce nonsense-mediated mRNA decay, form a complex. This complex is then degraded by endoribonucleases such as SMG6 and exoribonucleases such as SMG5 and SMG7, leading to the degradation of mRNA containing a premature termination codon (PTC). (premature termination codon; PTC) are degraded.

Recently, mechanisms that rapidly degrade mRNA of genes associated with specific diseases, such as microRNA, to suppress gene expression have been proposed as potential therapeutic agents, leading to significant interest and research in the development of disease therapies through mRNA stability regulation.

### Technical Problem

One objective of the present invention is to provide a circular RNA (Circular RNA) that binds complementarily to the 3' untranslated region of target mRNA and degrades the target mRNA.

Another objective of the present invention is to provide a pharmaceutical composition for the prevention or treatment of cell death-related diseases, comprising the circular RNA as an active ingredient.

Another objective of the present invention is to provide a pharmaceutical composition for the prevention or treatment of autophagy-mediated diseases, comprising the circular RNA as an active ingredient.

However, the technical problem to be achieved by the present invention is not limited to the above-mentioned problems, and other problems that are not mentioned will be clearly understood by a skilled person in the art from the following description.

### Solution to Problem

Various embodiments of the present invention are described with reference to the accompanying drawings. In the following description, various specific details, such as specific forms, compositions, and processes, are described for a complete understanding of the present invention. However, specific embodiments may be implemented without one or more of these specific details or in combination with other known methods and forms. In other examples, known processes and manufacturing techniques are not described in detail to avoid making the present invention unnecessary or ambiguous. A reference throughout this specification to a single embodiment means that the special features, forms, compositions, or characteristics described in connection with that embodiment are included in one or more embodiments of the present invention. Therefore, the situations of embodiments described at various locations throughout this specification do not necessarily represent the same embodiments of the invention. Additionally, special features, forms, compositions, or characteristics may be combined in any suitable manner in one or more embodiments.

In the present invention, the binding of circular RNA and mRNA was induced based on the nucleotide sequence. It was revealed that when circular RNA with an EJC binds to mRNA, the NMD phenomenon is induced, leading to the degradation of mRNA, and this mechanism was named circNMD. Additionally, the circNMD phenomenon may occur not only through the nucleotide sequence-based mechanism described above but also through other various methods that induce binding.

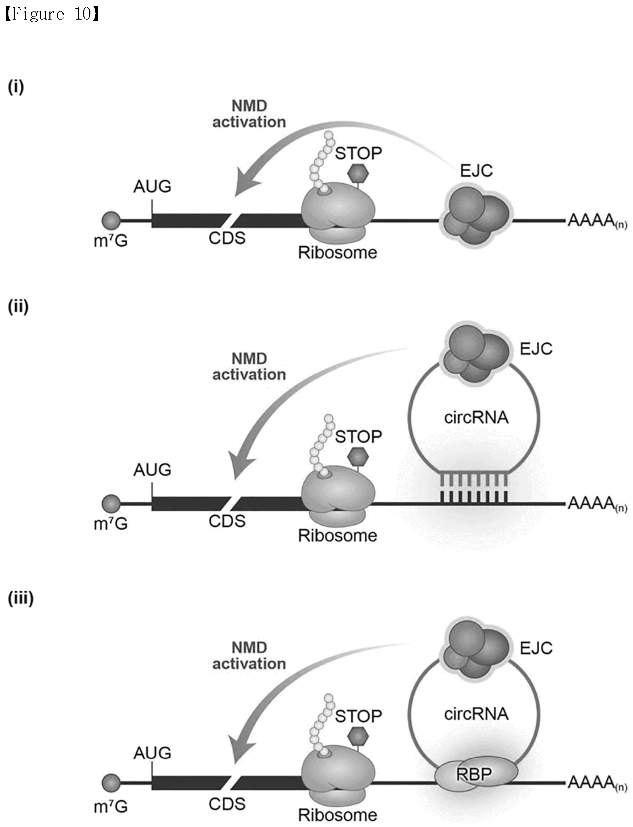

First, circNMD is induced by binding the circular RNA and mRNA together through the interaction between RNA-binding proteins that bind to each of the circular RNA and mRNA. Specifically, as shown in Figure 10(i-iii), the exon junction complex (exon junction complex; EJC) positioning the target mRNA at the 3' UTR is crucial for the circNMD phenomenon, so even if the circular RNA is not complementary to the target mRNA, the circNMD phenomenon can occur through various interactions between RNA-binding proteins that bind to both the circular RNA and the target mRNA, thereby activating NMD.

Second, In vitro, circular RNA is synthesized and the exon junction complex (EJC) is artificially positioned to synthesize circular RNA containing the exon junction complex (EJC). The circular RNA containing the exon junction complex (EJC) is reintroduced into cells to induce circNMD. Methods for artificially positioning the EJC include using RNA aptamers that can bind to EJC components. Alternatively, expressing EJC component proteins fused with dCas13 and guide RNA that can form base pairs with the circular RNA can position the EJC on the circular RNA.

### 1. Circular RNA (Circular RNA) that binds complementarily to the target mRNA and degrades the target mRNA

In one embodiment of the present invention, a circular RNA (Circular RNA) that binds complementarily to the target mRNA and degrades the target mRNA is provided.

In the present invention, the term "circular RNA (Circular RNA)" refers to RNA that exists in a circular form, unlike linear mRNA (linear RNA) with the well-known 5' cap and 3' poly(A) tail structures, and is referred to as circular RNA (circular RNA). Circular RNA is formed through back splicing rather than conventional splicing and, unlike linear mRNA, lacks a 5' cap and 3' poly(A) tail, conferring resistance to exoribonucleases and thereby enhancing stability.

In this invention, the term "target mRNA" refers not only to mRNA associated with specific diseases but also to RNA that encodes specific genes. Target RNA includes tRNA (transfer RNA), mRNA (messenger RNA), rRNA (ribosomal RNA), various small noncoding RNAs, long noncoding RNAs, RNA from viruses/bacteria, or RNA produced by pathogens, or RNA created through artificial genetic recombination methods, such as ribo-polynucleotides, but is not limited to these. In particular, it may include RNA that serves as a candidate substance useful in biotechnology, medicine, pharmacology, or substances that induce biological effects. Specifically, the target RNA may be naturally occurring sequences or newly modified sequences, such as the MS2 RNA of bacteriophages, the trans-activation response element (TAR) RNA of HIV viruses, or the iron-responsive element (IRE) RNA of humans or its variants, but is not limited to these.

The "target RNA" of the present invention may be used interchangeably with "target RNA."

In some embodiments of the present invention, the circular RNA may have a sequence that forms a complementary bond with the target mRNA. In this case, the complementary bond may optionally include at least one mismatch bond. For example, the circular RNA may have a sequence that hybridizes with or forms a complementary bond with the target mRNA, wherein the complementary bond may include 0 to 5 mismatches. Alternatively, the circular RNA may have a nucleotide sequence that is at least 70% complementary to the target mRNA.

As an embodiment, the circular RNA may be at least 70% to 75%, at least 70% to 80%, at least 70% to 85%, at least 70% to 90%, at least 70% to 95%, at least 70% to 100%, at least 75% to 80%, at least 75% to 85%, at least 75% to 90%, at least 75% to 95%, or at least 75% to 100% complementary to the target mRNA. Alternatively, the circular RNA may be at least 80% to 85%, at least 80% to 90%, at least 80% to 95%, at least 80% to 100%, at least 85% to 90%, at least 85% to 95%, or at least 85% to 100% complementary sequences. or the circular RNA may have a sequence that is at least 90% to 95%, at least 90% to 100%, or at least 95% to 100% complementary to the target mRNA. Or, the circular RNA may have a sequence that is at least 70%, 71%, 72%, 73%, 74%, 75%, 76%, 77%, 78%, 79%, 80%, 81%, 82%, 83%, 84%, 85%, 86%, 87, 88, 89, 90, 91, 92, 93, 94, 95, 96, 97, 98, 99, or 100% complementary to the target mRNA.

In other embodiments of the present invention, a circular nucleotide is provided that includes a sequence synthesized in vitro to bind complementarily to the untranslated region (UTR) of the target mRNA to remove the target mRNA, or a sequence generated through back-splicing within cells. The circular nucleotides provided in the present invention can be synthesized in vitro and introduced into cells, or expressed within cells through back-splicing, and can bind to the 3' untranslated region of the target mRNA to remove the target mRNA.

In another embodiment of the present invention, the nucleotides are provided as RNA or DNA encoding the nucleotides.

In another embodiment of the present invention, the circular RNA or circular nucleotides provide circular RNA that binds complementarily to the 3' untranslated region (UTR) of the target mRNA.

In another embodiment of the present invention, the nucleotides are provided as nucleotides that are complementary to the target mRNA and are located at least 100 nucleotides away from the stop codon of the target mRNA. In another embodiment of the present invention, nucleotides that bind complementarily to the target mRNA at a distance of 150 nucleotides or more, 200 nucleotides or more, 250 nucleotides or more, or 300 nucleotides or more from the stop codon are provided. If the nucleotides bind complementarily too close to the stop codon, they cannot recognize the exon junction complex located 50-55 nucleotides downstream of the mRNA stop codon, thereby preventing a reduction in mRNA levels.

In the present invention, the term "3' untranslated region (UTR)" refers to the portion of messenger RNA (mRNA) following the translation termination codon, and the 3' untranslated region often includes regulatory regions that influence post-transcriptional gene expression. Regulatory regions within the 3'-UTR can influence polyadenylation, translation efficiency, localization, and mRNA stability, and the 3'-UTR contains binding sites for both regulatory proteins and microRNAs (miRNAs). miRNA can reduce the gene expression of various mRNAs by binding to specific sites within the 3'-untranslated region, thereby inhibiting translation or directly inducing the degradation of transcripts. The circular RNA according to the present invention can reduce the gene expression of various mRNAs by binding to the 3'-untranslated region, thereby inhibiting translation or directly inducing the degradation of transcripts. The 3'-untranslated region also contains a silencing region that binds to inhibitory proteins to suppress mRNA expression. The 3'-untranslated region may also include sequences that recruit proteins to bind mRNA to the cytoskeleton, transport it to or from the cell nucleus, or perform other types of localization.

In another embodiment of the present invention, a circular RNA is provided that enables the exon junction complex (EJC) to bind to the target mRNA through binding to the circular RNA.

In another embodiment of the present invention, the circular nucleotides are provided to bind to exon junction complex (EJC) factors.

In the present invention, the "exon junction complex (EJC)" is a protein complex that binds to the junction between two exons that are joined together during RNA splicing. The exon junction complex significantly influences translation, surveillance, localization of spliced mRNA, and m6A methylation. The exon junction complex first binds to mRNA during splicing and is then transported to the cytoplasm. There, the exon junction complex plays a crucial role in post-transcriptional regulation of mRNA. The exon junction complex consists of a stable heteroquaternary core that serves as a binding platform for other components required for the mRNA pathway. At the core of the exon junction complex are the protein eIF4A3 (DEAD-box RNA helicase), which is bound to an adenosine triphosphate (ATP) analog, and additional proteins MAGOH, MLN51, and Y14.

In another embodiment of the present invention, the exon junction complex provides a nucleotide selected from the group consisting of eIF4A3, MNL51, Y14, and MAGOH.

That is, within eukaryotic cells, when mRNA is synthesized from DNA through the transcription process, the stability of the mRNA is determined by various factors. The stability of mRNA is influenced by various elements, including its structure, nucleotide sequence, mRNA-binding proteins, and internal RNA modifications, and this stability ultimately regulates gene expression.

The present invention relates to a new method for rapidly degrading target mRNA. When circular RNA (EJC-loaded circular RNA) or circular nucleotides bound to an exon junction complex (EJC) bind to the 3' untranslated region of target mRNA, the mRNA becomes a target for nonsense-mediated mRNA decay (NMD) and is rapidly degraded, thereby inducing a reduction in the expression of the corresponding gene.

In another embodiment of the present invention, the exon junction complex provides circular RNA that binds to the circular RNA.

In the present invention, a circular RNA having an exon junction complex and a sequence that can bind complementarily to the 3' UTR of the reporter mRNA, which can experimentally verify RNA stability, was produced. Specifically, a reporter system was constructed by artificially binding circular RNA containing an exon-splice complex to the 3' untranslated region of target mRNA to rapidly degrade the mRNA, and this system was introduced into HeLa cell lines. Subsequently, the quantity of mRNA was quantitatively observed using quantitative real-time polymerase chain reaction (qRT-PCR) experimental methods. As a result, it was found that the target mRNA was rapidly degraded by the circular RNA bound to the 3' untranslated region of the target mRNA.

In another embodiment of the present invention, the exon junction complex provides circular RNA selected from a group comprising eIF4A3, MNL51, RNPS1, Y14, SRm160, Aly/REF, Magoh, and DEK.

In another embodiment of the present invention, the circular nucleotides are provided to induce circNMD.

In the present invention, the term "circNMD" refers to the mechanism by which the NMD phenomenon is induced when circular RNA containing an EJC binds to mRNA, leading to the degradation of mRNA. Specifically, in the present invention, the binding of circular RNA and mRNA was induced based on their nucleotide sequences. This revealed the mechanism by which the binding of circular RNA to mRNA induces the NMD phenomenon, leading to mRNA degradation, and this mechanism was named circNMD. Additionally, the circNMD phenomenon may occur not only through nucleotide sequence-based mechanisms but also through other various methods that induce binding.

First, circNMD is induced by binding the circular RNA and mRNA together through the interaction between RNA-binding proteins that bind to each of the circular RNA and mRNA. Specifically, as shown in Figure 10(i-iii), positioning the exon junction complex (exon junction complex; EJC) positioning the target mRNA at the 3' UTR is crucial for the circNMD phenomenon, so even if the circular RNA is not complementary to the target mRNA, the circNMD phenomenon can occur through various interactions between RNA-binding proteins that bind to both the circular RNA and the target mRNA, thereby activating NMD.

Second, In vitro, circular RNA is synthesized and the exon junction complex (EJC) is artificially positioned to synthesize circular RNA containing the exon junction complex (EJC). The circular RNA containing the exon junction complex (EJC) is reintroduced into cells to induce circNMD. Methods for artificially positioning the EJC include using an RNA aptamer that can bind to EJC components. Alternatively, EJC component proteins fused with dCas13 and guide RNA capable of forming base pairs with the circular RNA can be co-expressed to position the EJC on the circular RNA.

In another embodiment of the present invention, the circNMD provides nucleotides that are subject to nonsense-mediated mRNA decay (NMD).

In another embodiment of the present invention, the circular RNA is provided, wherein the target mRNA degradation is caused by nonsense-mediated mRNA decay (NMD).

In the present invention, the "nonsense-mediated mRNA decay (NMD)" refers to proteins such as exon junction complex (EJC) factors and nonsense-mediated mRNA decay factors such as UPF1, UPF2, PNRC2, etc., which can induce nonsense-mediated mRNA decay. EJC) factors and proteins such as UPF1, UPF2, and PNRC2, which can induce nonsense-mediated mRNA decay, form a complex and are degraded by endoribonucleases such as SMG6, exoribonucleases such as SMG5, and SMG7, resulting in the degradation of defective mRNA containing a premature termination codon (premature termination codon; PTC) are degraded.

In another embodiment of the present invention, the circNMD provides nucleotides that are induced by binding the circular nucleotides and target mRNA through mutual binding between RNA-binding proteins that bind to the circular nucleotides and the target mRNA, respectively.

In another embodiment of the present invention, a composition for removing target nucleotides is provided, comprising the circular nucleotides.

Another embodiment of the present invention provides a composition for removing target nucleotides, wherein the nucleotides are RNA or DNA encoding RNA.

In one embodiment of the present invention, a method for removing target nucleotides is provided, comprising the circular nucleotides described above as an active ingredient. The nucleotides are produced extracellularly, administered to cells, expressed within cells, or administered to a target, thereby effectively removing the target nucleotides.

In one embodiment of the present invention, the nucleotides are RNA or DNA encoding the same, and a method for removing the target nucleotides is provided.

### 2. A pharmaceutical composition for the prevention or treatment of cell death-related diseases, comprising the circular RNA as an active ingredient.

In one embodiment of the present invention, a pharmaceutical composition for the prevention or treatment of cell death-related diseases, comprising circular RNA as an active ingredient, is provided.

In one embodiment of the present invention, a pharmaceutical composition for the prevention or treatment of cell death-related diseases is provided, comprising circular nucleotides as an active ingredient.

The circular RNA provided in the present invention binds complementarily to the BCL2L11 gene related to cell death, thereby inhibiting the expression of the protein and reducing cell death.

The protein encoded by the BCL2L11 gene in the present invention belongs to the BCL-2 protein family. Members of the BCL-2 family form heterodimers or homodimers and act as anti-apoptotic regulators or pro-apoptotic regulators involved in various cellular activities. The protein encoded by this gene contains the Bcl-2 homology domain 3 (BH3), which interacts with other members of the BCL-2 protein family, including BCL2, BCL2L1/BCL-X(L), and MCL1, and acts as an apoptosis activator. The expression of this gene can be induced by nerve growth factor (NGF) as well as by the forkhead transcription factor FKHR-L1 (FoxO3a), suggesting a role for this gene in neuronal and lymphocyte apoptosis. Transgenic studies in mouse counterparts suggest that this gene functions as an essential initiator of apoptosis in thymocyte negative selection.

In the present invention, the term "apoptosis" refers to the natural death of cells through a predetermined series of steps. Apoptosis, also known as programmed cell death, is a type of programmed cell death observed in multicellular organisms. Cell suicide is defined as the death of a cell due to changes in its morphology and internal biochemical processes. This process culminates in cell swelling and rupture, changes in the cell membrane, nuclear fragmentation, chromatin condensation, chromosome fragmentation, and the eventual ingestion and processing of the cell by other cells. In contrast to necrosis, which is caused by sudden damage to cells, apoptosis does not harm the organism and is beneficial to the life cycle. The formation of fingers and toes, each consisting of 10 digits, during the differentiation process of a human embryo is a representative example of apoptosis. Many proteins in the cytoplasm are involved in the apoptosis pathway (CAD proteins), and these proteins are normally bound to inhibitors but are activated by apoptosis-inducing substances.

In other embodiments of the present invention, the cell death-related diseases include autoimmune diseases, graft-versus-host disease, organ transplant rejection, asthma, atopy, or acute or chronic inflammatory diseases, and a pharmaceutical composition is provided.

To investigate whether the same phenomenon occurs in mRNA and circular RNA present in actual cells, microarray and RNA in situ conformation sequencing (RIC-seq) data analysis were performed to identify mRNA and circular RNA candidates with a high likelihood of binding within cells. Among these, BCL2L11 mRNA was found to reduce mRNA stability through binding with circular RNA provided in this invention, such as hsa_circ_0002082 or hsa_circ_0008496, thereby affecting cellular functions such as apoptosis.

Neurons differ from other cells in the body in that approximately half of the cells generated die during the developmental stage. This is believed to result from competition for limited trophic factors, leading to the selective survival of cells that form accurate synaptic connections with target neurons at synaptic terminals. Additionally, studies using cultured neurons or in vivo brain observations have reported that many neurons die under deteriorated survival conditions (e.g., external stress) in the adult brain, and in many cases, this has been confirmed to be due to cell death mechanisms. Therefore, the circular RNA provided in this invention can exert preventive and therapeutic effects by inhibiting cell death in the aforementioned neurodegenerative or neuroinflammatory diseases that occur through such cell death mechanisms. The aforementioned neurodegenerative or neuroinflammatory diseases include stroke, dementia, Alzheimer's disease, Parkinson's disease, Huntington's disease, Niemann-Pick disease, prion disease, Creutzfeldt-Jakob disease, frontotemporal dementia, Lewy body dementia, amyotrophic lateral sclerosis (ALS), paraneoplastic syndrome, cortical basal degeneration, multiple system atrophy, progressive supranuclear palsy, autoimmune diseases of the nervous system, spinocerebellar ataxia, inflammatory and neuropathic pain, cerebrovascular diseases, spinal cord injury, and tauopathy, but is not limited to these.

In the present invention, the term "autoimmune disease" refers to a disease manifested by autoimmune responses. In other words, it is a condition where the immune system mistakenly reacts to normal chemicals and certain cells in the body. In simpler terms, it is an immune response error that identifies healthy cells as harmful and attacks them, leading to cell death. The circular RNA provided in the present invention can effectively prevent or treat the aforementioned autoimmune diseases that cause cell death in healthy cells by inhibiting cell death. The aforementioned autoimmune diseases include rheumatoid arthritis, type 1 diabetes (insulin-dependent), systemic lupus erythematosus, Crohn's disease, psoriasis, and others.

### 3. Pharmaceutical composition for the prevention or treatment of autophagy-mediated diseases containing the circular RNA as an active ingredient

In an embodiment of the present invention, a pharmaceutical composition for the prevention or treatment of autophagy-mediated diseases containing the circular RNA as an active ingredient is provided.

In the present invention, "autophagy" refers to a biological process occurring within eukaryotic cells, which maintains cellular homeostasis by destroying and recycling cellular components. It is a term that describes the overall process in which cellular components are aggregated into autophagosomes, which are double-membrane structures, and then degraded by lysosomes.

In this invention, circular RNA containing sequences that bind to naturally occurring ATG5 or ATG7 mRNA within cells was produced and artificially overexpressed in cells, resulting in a rapid decrease in the stability of the corresponding mRNA. Through this process, it was confirmed that the cellular function of autophagy was inhibited.

Autophagy is an intracellular degradation mechanism that maintains cellular homeostasis by degrading unnecessary or non-functional proteins, organelles, and nutrients within the cell via lysosomes in response to various external stress environments. Autophagy, which plays a crucial role in maintaining cellular homeostasis, has been shown to be closely associated with various diseases such as cancer, neurodegenerative diseases, diabetes, and aging through studies using animal models and human-derived disease models. Therefore, regulating autophagy has emerged as an important target for developing new drug mechanisms and therapeutic strategies for treating related diseases. Particularly, autophagy has been reported to exhibit functions that suppress tumor formation in normal cells during cancer development and growth, and conversely, to promote tumor growth and resistance to anticancer therapy once cancer has formed. As a result, understanding the precise regulatory mechanisms of autophagy and developing regulatory molecules tailored to the stage of cancer development has garnered significant attention. Additionally, recent studies have revealed that cancer stem cells (CSCs) rely heavily on autophagy activity during their maintenance, generation, and differentiation processes, unlike other cells. This has led to the expectation that regulating autophagy could provide a new paradigm for anticancer therapy.

In another embodiment of the present invention, the autophagy-mediated disease is selected from a group consisting of one or more of cancer, brain and nervous system diseases, diabetes, myocardial cell death diseases, obesity, and viral infections, and a pharmaceutical composition is provided.

In the present invention, myocardial cell death disease refers to cardiovascular diseases caused by the death of myocardial cells. This is a general term for diseases of the circulatory system, including hypertension, ischemic heart disease, angina pectoris, myocardial infarction, atherosclerosis, stroke, and arrhythmia, which are caused by factors such as age, diabetes, obesity, and smoking. Autophagy is an essential cellular mechanism for maintaining normal cardiovascular function; however, excessive autophagy can lead to the aforementioned diseases.

In this invention, cancer refers to malignant tumors that grow abnormally due to excessive growth. Examples of cancer include gastric cancer, breast cancer, lung cancer, liver cancer, blood cancer, bone cancer, pancreatic cancer, skin cancer, head or neck cancer, cutaneous or intraocular melanoma, uterine sarcoma, ovarian cancer, rectal cancer, anal cancer, colon cancer, fallopian tube carcinoma, endometrial carcinoma, cervical cancer, small intestine cancer, endocrine cancer, thyroid cancer, parathyroid cancer, adrenal cancer, soft tissue tumor, urethral cancer, prostate cancer, bronchogenic cancer, and bone marrow tumor, and is not limited thereto.

In another embodiment of the present invention, the brain and nervous system disease is a neurodegenerative disease or a neuroinflammatory disease, and a pharmaceutical composition is provided.

In another embodiment of the present invention, the neurodegenerative disease or neuroinflammatory disease is stroke, dementia, Alzheimer's disease, Parkinson's disease, Huntington's disease, Niemann-Pick disease, Creutzfeldt-Jakob disease, frontotemporal dementia, Lewy body dementia, amyotrophic lateral sclerosis (ALS), paraneoplastic syndrome, cortical basal degeneration, multiple system atrophy, progressive supranuclear palsy, neurological autoimmune diseases, spinocerebellar ataxia, inflammatory and neuropathic pain, cerebrovascular diseases, spinal cord injury, and tauopathy, comprising at least one selected from the group.

Furthermore, in the present invention, "treatment" includes any act that improves or benefits the symptoms of the disease using the pharmaceutical composition of the present invention, without limitation.

Furthermore, without limitation, the methods for preventing or treating the aforementioned diseases may include combination therapy that further comprises administering a compound or substance having therapeutic activity against one or more of the aforementioned diseases.

In the present invention, the term "combination" should be understood to mean simultaneous, individual, or sequential administration. In the case of sequential or individual administration, the interval between the administration of the second component should be such that the beneficial effects of the combination are not lost.

In the present invention, the pharmaceutical composition may be characterized by being in the form of capsules, tablets, granules, injections, ointments, powders, or beverages, and the pharmaceutical composition may be characterized by being intended for use in humans.

In the present invention, the pharmaceutical composition is not limited to the above, but may be formulated and used in the form of oral dosage forms such as suspensions, granules, capsules, tablets, aqueous suspensions, etc., topical agents, suppositories, and sterile injection solutions, each prepared by conventional methods. The pharmaceutical composition of the present invention may include a pharmaceutically acceptable carrier. A pharmaceutically acceptable carrier may include, for example, in the case of oral administration, binders, disintegrants, diluents, excipients, solubilizers, dispersants, stabilizers, suspension agents, colorants, and flavorings, while for injectable formulations, buffering agents, preservatives, analgesics, solubilizers, emulsifiers, and stabilizers may be mixed, and for topical formulations, vehicles, excipients, lubricants, and preservatives may be used. The dosage form of the pharmaceutical composition of the present invention can be manufactured in various ways by mixing with the pharmaceutically acceptable carriers described above. For example, for oral administration, it can be manufactured in the form of tablets, troches, capsules, elixirs, suspensions, syrups, or wafers. For injectable formulations, it can be manufactured in the form of single-dose ampoules or multi-dose formulations. Additionally, it can be formulated as solutions, suspensions, tablets, capsules, or sustained-release formulations.

Examples of suitable carriers, excipients, and diluents for formulation include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, or mineral oil. Additionally, fillers, anti-caking agents, lubricants, humectants, flavorings, emulsifiers, preservatives, etc., may be further included.

The administration routes of the pharmaceutical composition of the present invention are not limited to the following, but may include direct administration to the eye, oral, intravenous, intramuscular, intra-arterial, intra-bone marrow, intra-dural, intra-cardiac, transdermal, subcutaneous, intra-abdominal, intra-nasal, intra-intestinal, local, sublingual, or rectal administration. Oral or non-oral administration is preferred. Direct administration into the eye is more preferable.

In the present invention, the term "non-oral" includes direct administration into the eye, or injection or infusion techniques such as subcutaneous, intradermal, intravenous, intramuscular, intra-articular, intra-synovial, intra-sternal, intra-meningeal, intra-lesional, and intracranial administration. The pharmaceutical compositions of the present invention may also be administered in the form of suppositories for rectal administration. Direct administration into the eye is preferred.

The "dosage and administration" of the composition of the present invention may vary depending on various factors, including the activity of the specific compounds used, age, weight, general health, gender, condition, administration time, administration route, excretion rate, drug combination, and the severity of the specific disease to be prevented or treated. The dosage of the pharmaceutical composition may vary depending on the patient's condition, weight, severity of the disease, drug form, administration route, and duration, but may be appropriately selected by a skilled practitioner. The dosage may range from 0.0001 to 50 mg/kg or 0.001 to 50 mg/kg per day. Administration may be once daily or divided into multiple doses. The above dosage does not limit the scope of the present invention in any way. The pharmaceutical composition according to the present invention may be formulated as tablets, capsules, capsules, liquids, gels, syrups, slurries, or suspensions.

### Advantageous Effects of Invention

The technology for promoting the degradation of target mRNA using circular RNA with an exon junction complex (EJC) has the advantages of being able to easily identify the desired target mRNA by modifying the binding sequence and using circular RNA, which is more stable than linear RNA and resistant to exoribonucleases present in cells. Therefore, this technology is expected to have significant utility in the development of therapeutic agents based on mRNA stability regulation.

Additionally, the exon junction complex (EJC) does not bind to the 3' UTR, and when it binds to a non-3' UTR region, NMD does not occur. However, after RNA is transcribed from DNA within cells, circular RNA is generated through back-splicing, and the exon junction complex (EJC) naturally binds to the circular RNA. If this circular RNA can bind to the 3'UTR of the target mRNA, NMD is activated, enabling the specific degradation of the target mRNA.

Specifically, first, by modifying the linear RNA binding site of circular RNA that possesses an exon junction complex (EJC), it is possible to target any desired mRNA without limitations on the target mRNA. In other words, as long as the nucleotide sequence of the mRNA is known, all mRNAs can be selectively targeted.

Second, using circular RNA enhances stability. Circular RNA is resistant to exoribonucleases, making it more stable than linear RNA. Therefore, using circular RNA offers superior stability for therapeutic agents compared to linear RNA.

Third, it can aid in the development of therapeutic technologies based on mRNA stability regulation. By using artificially created circular RNA to specifically degrade target mRNA, it is possible to develop treatments for various diseases.

### Brief Description of Drawings

Figure 1a shows the experimental schematic diagram of Example 1-1.
Figure 1b shows experimental results demonstrating that the amount of linear reporter mRNA decreases when circular effector RNA (circular effector RNA) that binds to linear reporter mRNA is present alongside it.
Figure 1c shows the experimental results of the same experiment as Figure 1b, but with the direction of the binding base sequences reversed.
Figure 2a shows the experimental schematic diagram of Example 1-2.
Figure 2b shows the relative amount of circular effector RNA (circular effector RNA) bound to linear reporter mRNA.
Figure 3a shows the experimental schematic diagram of Example 2.
Figure 3b shows the experimental results indicating the changes in the amount of each linear reporter mRNA when the circular effector RNA is present with each linear reporter mRNA bound to different regions.
Figure 4a shows the experimental schematic diagram of Example 3.
Figure 4b shows the Western blotting experimental results of various exon junction complex (EJC) factors binding to purified reporter mRNA.
Figure 4c shows the Western blotting results confirming that the purified reporter mRNA was immunoprecipitated with eIF4A3.
Figure 4d shows the amount of circular effector RNA bound when immunoprecipitated with eIF4A3.
Figure 4e shows the amount of linear reporter mRNA bound when immunoprecipitated with eIF4A3.
Figure 5a shows the experimental results observing changes in the amount of linear reporter mRNA when Nonsense-mediated mRNA decay (NMD) factors were reduced.
Figure 5b shows the experimental results observing changes in the stability of linear reporter mRNA upon reduction of NMD key factors.
Figure 5c shows the experimental results obtained by reversing the direction of the base sequence while reducing the stability of linear reporter mRNA in response to the reduction of NMD key factors.
Figure 6a shows a schematic diagram of the microarray experiment conducted in Example 5.
Figure 6b is a Venn diagram showing the number of linear RNA and circular RNA identified through microarray analysis.
Figure 6c shows the binding pairs between mRNA and circular RNA identified through RIC-seq.
Figure 7a shows the experimental results indicating the amount of target BCL2L11 mRNA when the amount of each circular RNA was reduced using siRNA.
Figure 7b is a graph quantitatively showing the percentage of cells undergoing apoptosis after reducing circular RNA.
Figure 7c shows an image of cells stained after reducing circular RNA.
Figure 8a shows a schematic diagram of the circular effector RNA produced in Example 6-2.
Figure 8b shows the amount of BCL2L11 mRNA after circular RNA was overexpressed.
Figure 8c is a graph quantitatively showing the percentage of cells undergoing apoptosis after circular RNA was overexpressed.
Figure 8d shows an image of cells stained after overexpressing circular RNA and inducing cell death.
Figure 9a is a schematic diagram of two circular effector RNAs designed to bind to the 3'UTR of ATG5 mRNA.
Figure 9b shows experimental results indicating that the amount of ATG5 mRNA decreases when the two types of circular effector RNA are expressed separately or together, and that the effect is further enhanced when both types are co-expressed.
Figure 9c shows experimental results demonstrating that as mRNA decreases, the amount of the corresponding protein also decreases, thereby inhibiting autophagy.
Figure 10 is a schematic diagram illustrating the present invention. Specifically, Figure 10(i) illustrates the previously known nonsense-mediated mRNA decay (NMD), and Figure 10(ii) illustrates the circular RNA provided by the present invention binding to the exon junction complex (exon junction complex; EJC) and the 3'-untranslated region (3'-UTR) of the target RNA, thereby activating nonsense-mediated mRNA decay (NMD). This phenomenon is referred to as circNMD in this specification. Figure 10(iii) is a diagram illustrating that the circular RNA provided by the present invention and the target mRNA are bound together by an RNA-binding protein common to both, thereby inducing circNMD.
Figure 11 shows that inhibiting the binding between linear reporter mRNA and circular effector RNA does not result in a decrease in the amount of linear mRNA. Specifically, Figure 11a is an experimental schematic diagram, and Figure 11b shows experimental results demonstrating that when ASO is treated to bind to linear reporter mRNA or circular effector RNA, linear reporter mRNA cannot bind to circular effector RNA, resulting in no reduction in the amount of linear reporter mRNA.
Figure 12 confirms that inhibiting the formation of normal circular effector RNA does not result in a decrease in linear reporter mRNA levels. Specifically, Figure 12a is an experimental schematic diagram, Figure 12b shows experimental results indicating that a reduction in linear reporter mRNA levels occurs only when circular effector RNA is formed, and Figure 12c shows experimental results obtained by reversing the direction of the binding sequence in the experiment described in Figure 12b.
Figure 13 confirms that the linear mRNA reduction phenomenon only occurs when the circular effector RNA binds at a position more than 100 nucleotides away from the stop codon. Specifically, Figure 13a is an experimental schematic diagram, and Figure 13b shows the experimental results confirming that a circular effector RNA must bind at a position more than 100 nucleotides away from the stop codon of the linear reporter mRNA when circular RNA is bound at various positions in the 3'UTR of the linear reporter mRNA.
Figure 14 shows that the reduction in linear reporter mRNA is more pronounced as the number of circular effector RNA binding sites increases. Specifically, Figure 14a is an experimental schematic diagram, and Figure 14b shows experimental results confirming that the amount of linear reporter mRNA decreases more significantly as the number of binding sites for circular effector RNA increases.
Figure 15 shows that the amount of linear reporter mRNA decreases more significantly as the number of circular effector RNA molecules binding to linear reporter mRNA increases. Specifically, Figure 15a is an experimental schematic diagram, and Figure 15b shows experimental results demonstrating that the amount of linear reporter mRNA decreases more significantly as the number of circular effector RNA molecules binding to linear reporter mRNA increases.
Figure 16 shows that inhibiting the binding between circular RNA and target mRNA increases cell death. Specifically, Figure 16a is a schematic diagram of ASOs specific to the binding sites of hsa_circ_0008496 and hsa_circ_0002082, Figure 16b is a graph showing the increased amount of *BCL2L11* mRNA after ASO treatment, Figure 16c is a graph quantitatively showing the percentage of cells undergoing cell death after ASO treatment, and Figure 16d is an image of cells undergoing cell death after ASO treatment.

### Detailed Description of Invention

Hereinafter, the present invention will be described in more detail by way of examples. These examples are only for describing the present invention in more detail, and it will be apparent to those skilled in the art that according to the gist of the present invention, the scope of the present invention is not limited by these examples.

### Examples

### Example 1. Reduction of linear reporter mRNA due to binding of circular RNA (Circular RNA)

### Example 1-1. Confirmation of the reduction in linear reporter mRNA levels due to circular RNA (circular RNA) binding

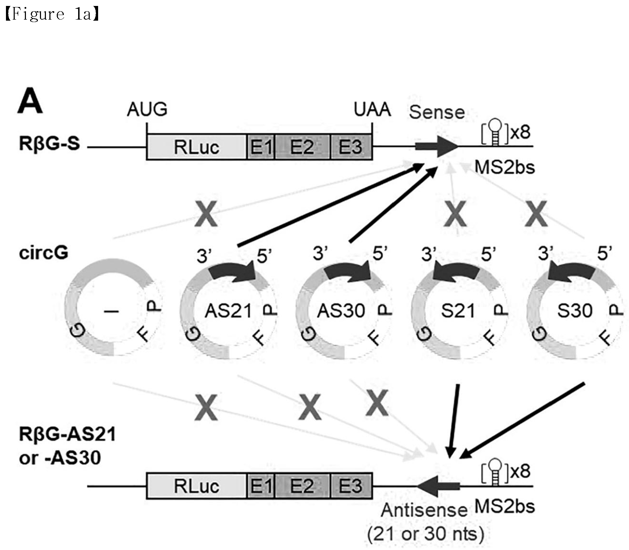

For nonsense-mediated mRNA decay (NMD) to occur, it is important that the exon junction complex (EJC) binds to the downstream region, i.e., the 3' untranslated region (UTR), of the stop codon. Considering this, as shown in Figure 1a, we designed linear reporter mRNA and circular effector RNA such that circular RNA, which possesses an exon junction complex due to back-splicing, can bind to the 3' UTR downstream of the stop codon of mRNA.

Specifically, circular effector antisense (AS21) and circular effector antisense AS30, which binds to 30 base sequences, were co-expressed with the linear reporter in HeLa cells, and the amount of each linear reporter mRNA was quantified.

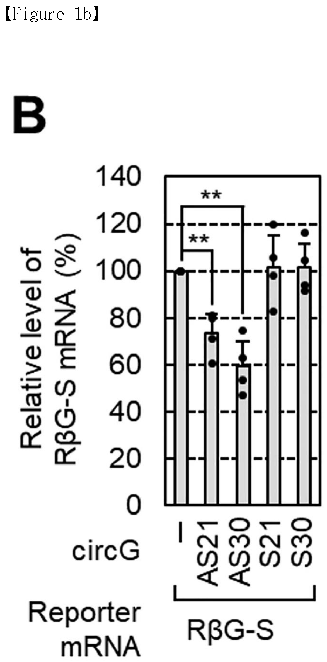

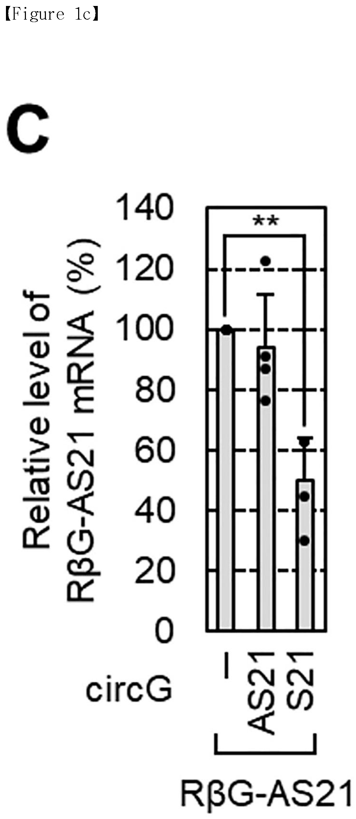

As a result, as shown in Figures 1b and 1c, when circular effector RNA capable of binding to linear reporter mRNA was co-expressed with linear reporter mRNA, a decrease in the amount of the aforementioned reporter mRNA was observed, whereas no change in its amount was observed when circular effector RNA with non-binding sequences was used.

### Example 1-2. Confirmation of binding between linear reporter mRNA and circular RNA (circular RNA)

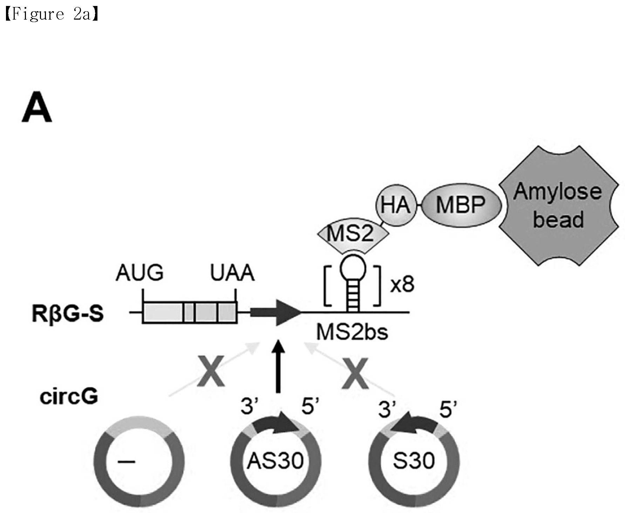

An experiment was conducted to confirm whether the circular effector RNA co-expressed directly binds to the linear reporter mRNA. As shown in Figure 2a, reporter mRNA containing an MS2 binding sequence (MS2bs) that can bind to MS2-HA-MBP protein, MS2-HA-MBP protein, and three circular effector RNAs with different binding sequences were simultaneously expressed in cells.

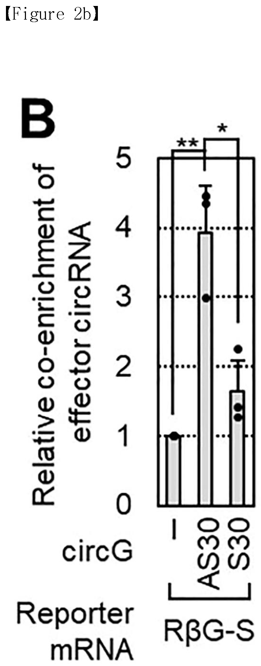

As shown in Figure 2b, MBP pull-down assay using amylose beads, which strongly bind to MBP, revealed that the circular effector RNA with a complementary nucleotide sequence to the linear reporter mRNA bound most strongly to the linear reporter mRNA.

### Examples 1-3. Confirmation of inhibition of binding between linear reporter mRNA and circular effector RNA using antisense oligonucleotides

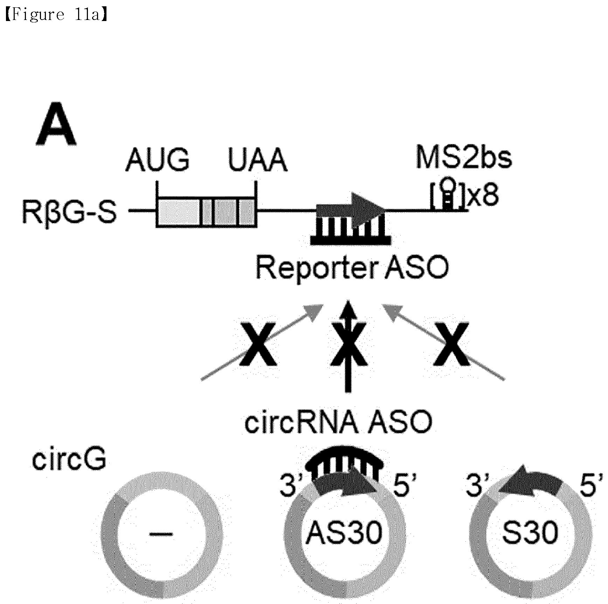

To inhibit the binding of linear reporter mRNA and circular effector RNA, antisense oligonucleotides (ASOs) that bind to either linear reporter mRNA or circular effector RNA were synthesized, as shown in Figure 11a.

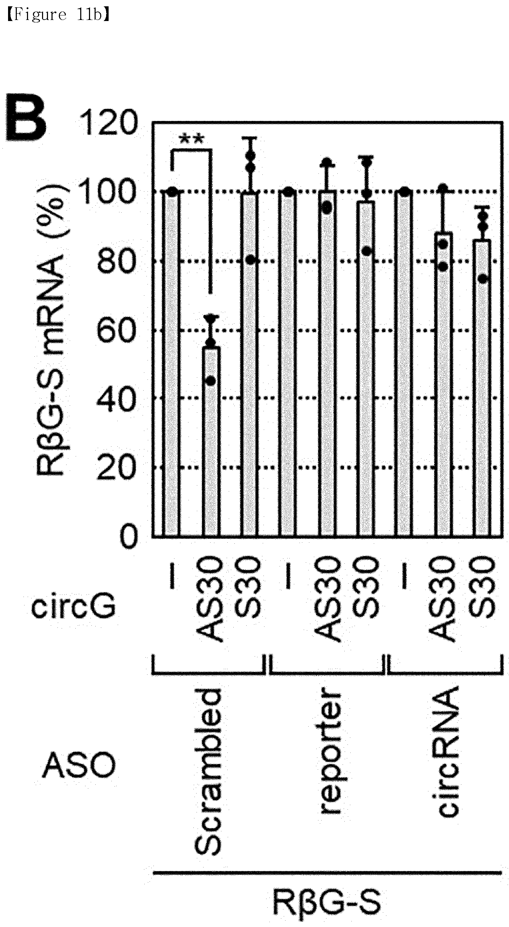

As shown in Figure 11b, it was confirmed that when the binding between linear reporter mRNA and circular effector RNA was disrupted using antisense oligonucleotides (ASOs), no reduction effect on linear reporter mRNA was observed.

### Example 1-4. Confirmation of the reduction effect on linear reporter mRNA depending on the form of effector RNA

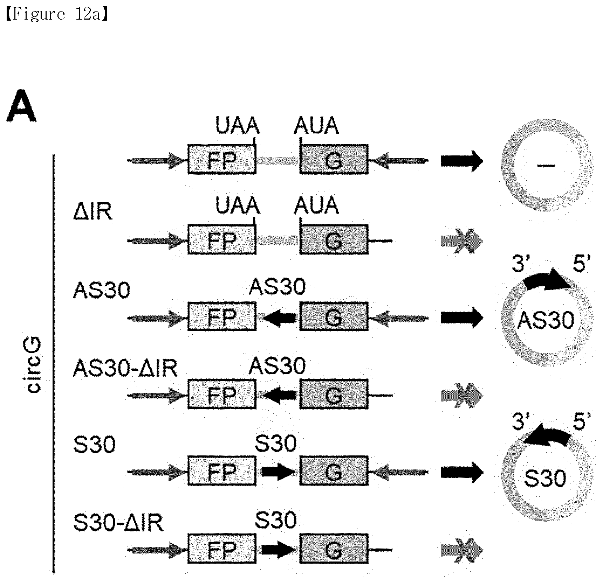

The backsplicing process that generates circular RNA occurs through repetitive sequences at both ends of the exon. Therefore, as shown in Figure 12a, we compared cases where normal circular effector RNA was generated with repetitive sequences at both ends of the exon and cases where one repetitive sequence was removed to prevent proper generation of circular effector RNA.

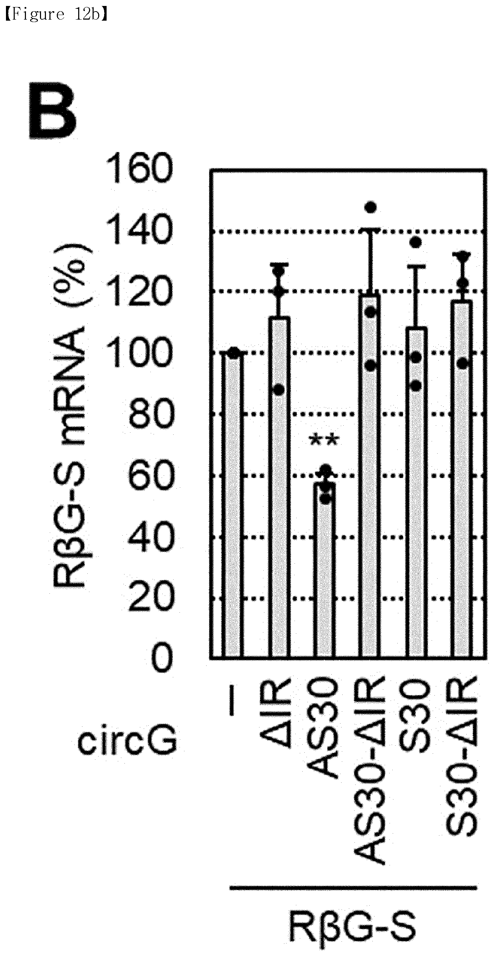

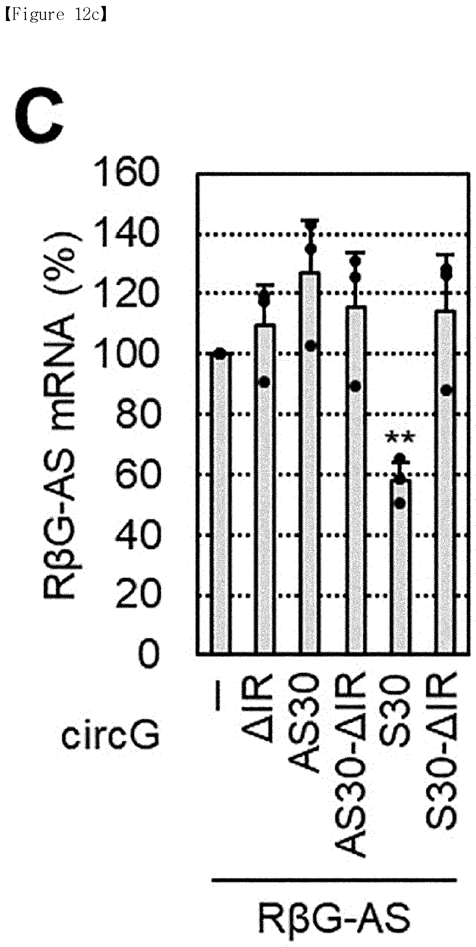

As shown in Figures 12b and 12c, when normally generated circular effector RNA binds to linear reporter mRNA, the amount of linear reporter mRNA decreases. However, when the repeat sequence is removed, preventing the normal generation of circular effector RNA, the amount of linear mRNA does not decrease.

### Example 2. Reduction of linear reporter mRNA depending on the location and number of the above-mentioned binding sites

### Example 2-1. Reduction in linear reporter mRNA specifically induced when the binding occurs only in the 3' UTR

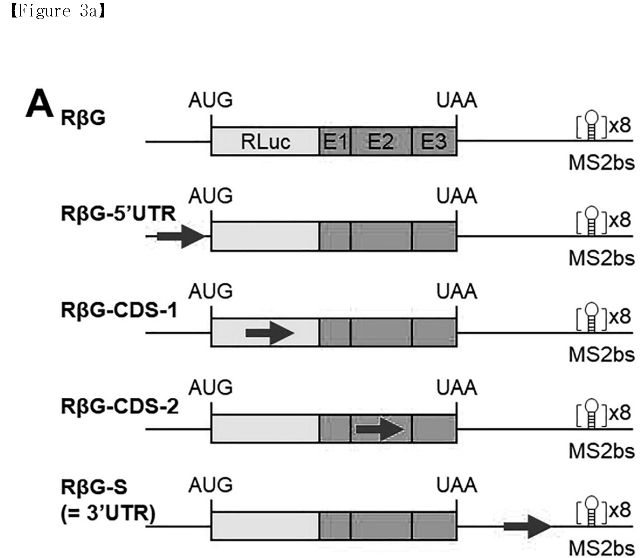

We sought to determine at which position of linear reporter mRNA the circular effector RNA binds to reduce the amount of linear reporter mRNA. As shown in Figure 3a, we created linear reporter mRNAs that allow the circular effector RNA (Circular effector RNA) to bind to the 5' untranslated region, coding sequence (CDS), and 3' untranslated region of the linear reporter mRNA.

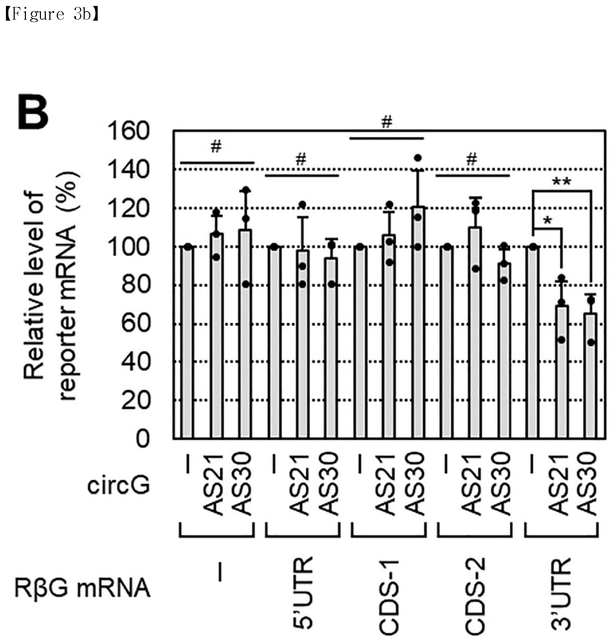

As shown in Figure 3b, we observed that the amount of linear reporter mRNA decreased only when the circular effector RNA bound to the 3' untranslated region of the linear reporter mRNA, and no such decrease was observed when it bound to other regions.

### Example 2-2. Confirmation of the linear reporter mRNA reduction effect specifically induced when the binding occurs at a location more than 100 nucleotides away from the stop codon

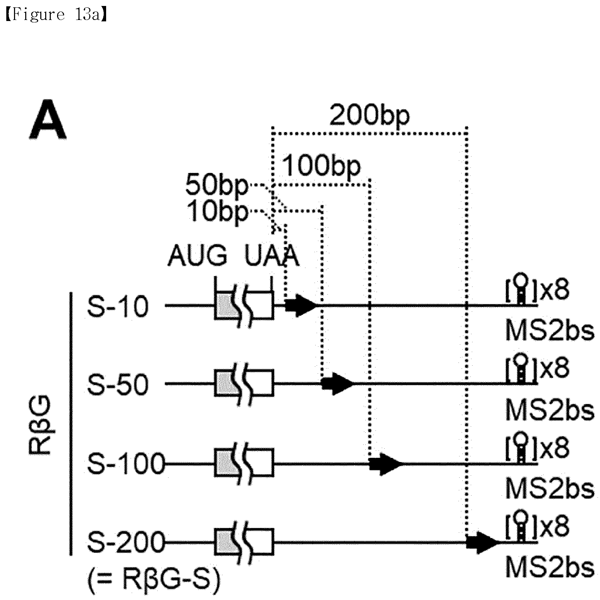

The nonsense-mediated mRNA degradation process occurs by recognizing the exon junction complex located 50-55 nucleotides downstream of the mRNA termination codon. Therefore, as shown in Figure 13a, we confirmed the reduction in linear reporter mRNA by having circular effector RNA bind to various regions of the 3'UTR of linear mRNA that contain exon junction complexes.

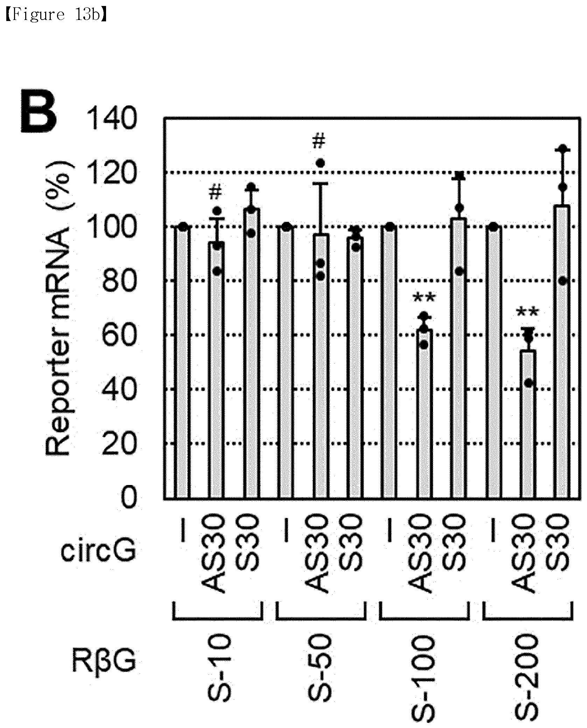

As shown in Figure 13b, it was observed that circular effector RNA must bind at least 100 nucleotides or more away from the termination codon of linear reporter mRNA to reduce the amount of linear reporter mRNA.

### Example 2-3. Confirmation of enhanced linear reporter mRNA reduction effect when increasing the number of binding sites

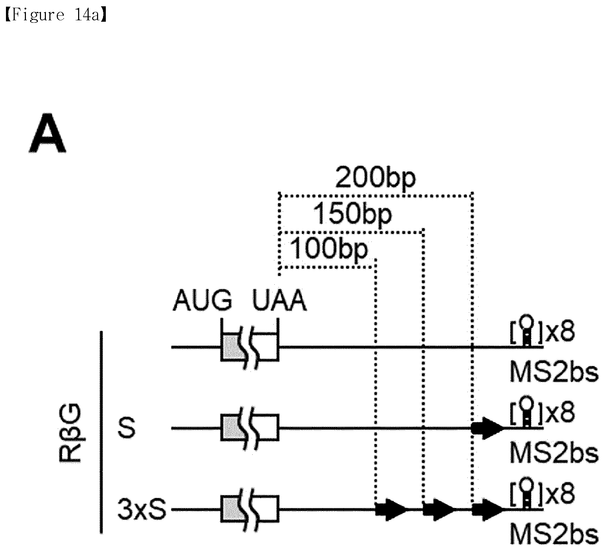

To confirm whether the reduction effect of linear reporter mRNA is enhanced by increasing the number of circular effector RNA binding sites in the 3'UTR of linear reporter mRNA, as shown in Figure 14a, binding sequences were inserted at positions 100, 150, and 200 nucleotides away from the stop codon.

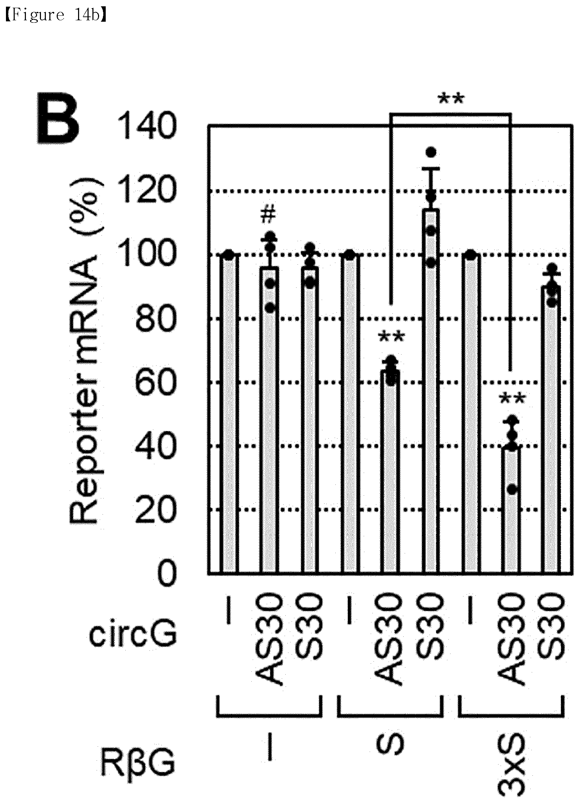

As shown in Figure 14b, it was observed that the reduction in linear reporter mRNA was more pronounced when there were three binding sites for circular effector RNA compared to when there was only one binding site.

### Example 2-4. Confirmation of enhanced linear reporter mRNA reduction effect depending on the number of circular effector RNAs binding to linear reporter mRNA

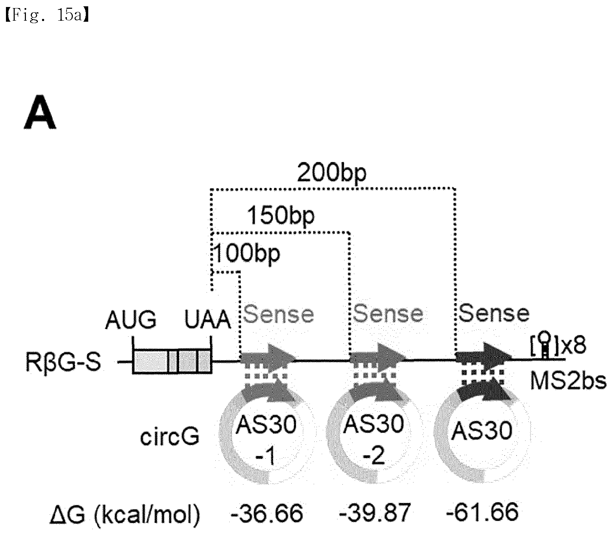

To confirm whether the reduction effect on linear reporter mRNA is enhanced when multiple circular effector RNAs binding to linear reporter mRNA are introduced, three circular effector RNAs binding to regions 100, 150, and 200 nucleotides away from the stop codon were created, as shown in Figure 15a.

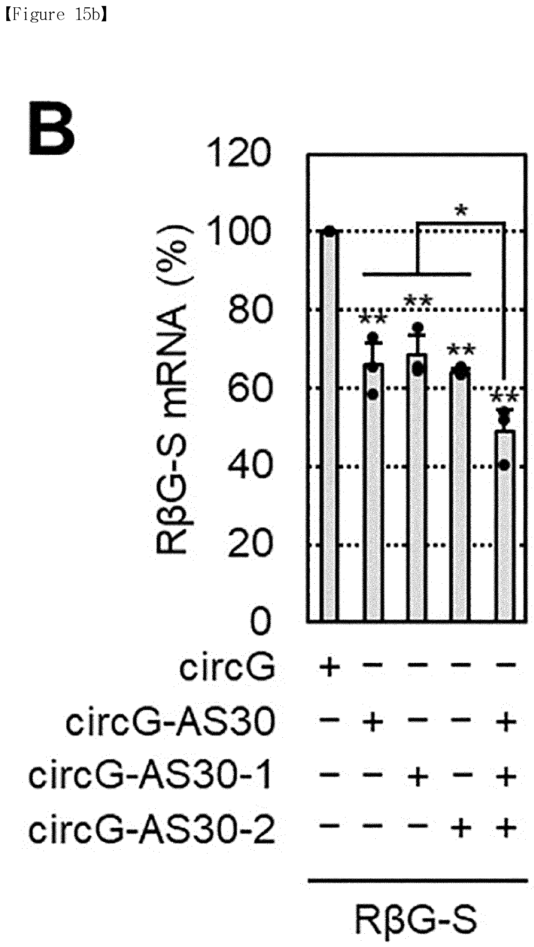

As shown in Figure 15b, it was observed that the amount of linear reporter mRNA decreased more significantly when all three circular effector RNAs were expressed together compared to when each circular effector RNA was expressed individually.

### Example 3. Binding between linear reporter mRNA and the exon junction complex (EJC) via circular effector RNA

### Example 3-1. Confirmation of binding between linear reporter mRNA and circular effector RNA via MBP pull-down analysis

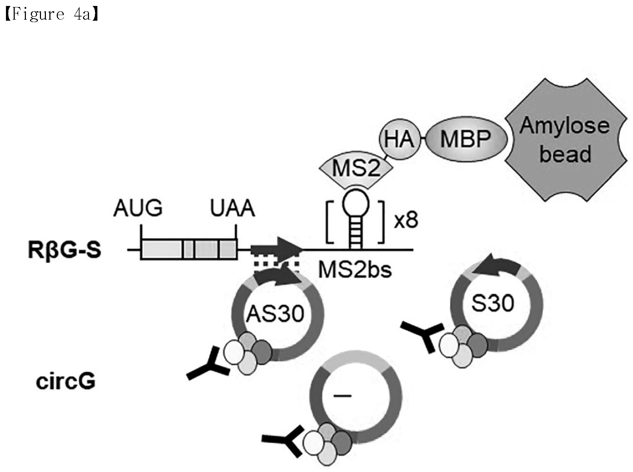

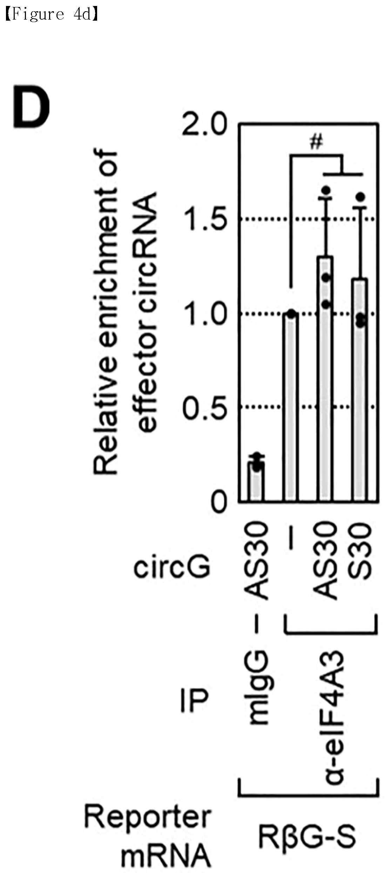

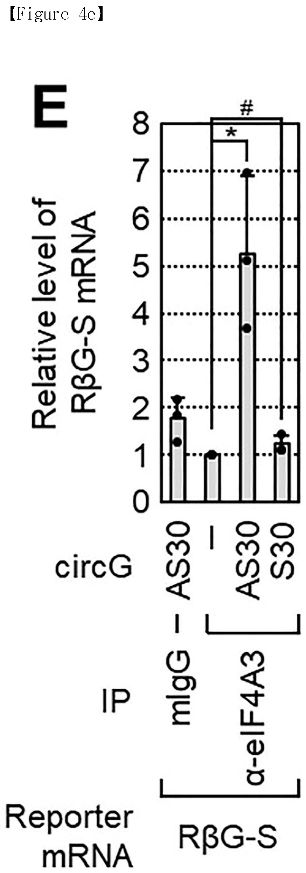

We sought to confirm whether circular effector RNA bound to linear reporter mRNA included the exon junction complex (EJC). Based on the experiment conducted in Figure 2a, we aimed to confirm the presence of exon junction complex (EJC) components in the linear reporter mRNA obtained through pull-down, as shown in Figure 4a, using Western blotting.

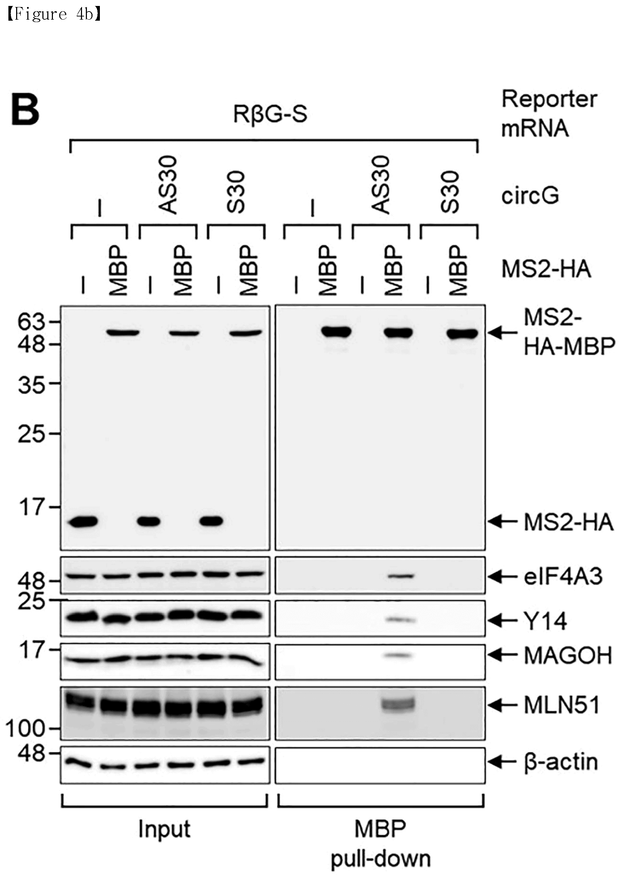

The experimental results showed that, as depicted in Figure 4b, the major EJC components eIF4A3, Y14, MAGOH, and MLN51 were present only when the circular effector RNA with a sequence binding to the linear reporter mRNA was co-expressed. This indicates that linear reporter mRNA binds to the exon junction complex (EJC) through its interaction with circular effector RNA.

### Example 3-2. Confirmation of the binding between linear reporter mRNA and circular effector RNA using exon junction complex (EJC) immunoprecipitation (IP)

To confirm whether circular RNA containing the exon junction complex (EJC) binds to linear reporter mRNA, as shown in Figure 4a, immunoprecipitation (IP) was performed using eIF4A3, one of the major factors of the exon junction complex (EJC), to quantify the amount of linear reporter mRNA bound to eIF4A3. (exon junction complex; EJC), one of the major components of the EJC, was performed to measure the amount of linear reporter mRNA bound to the EJC.

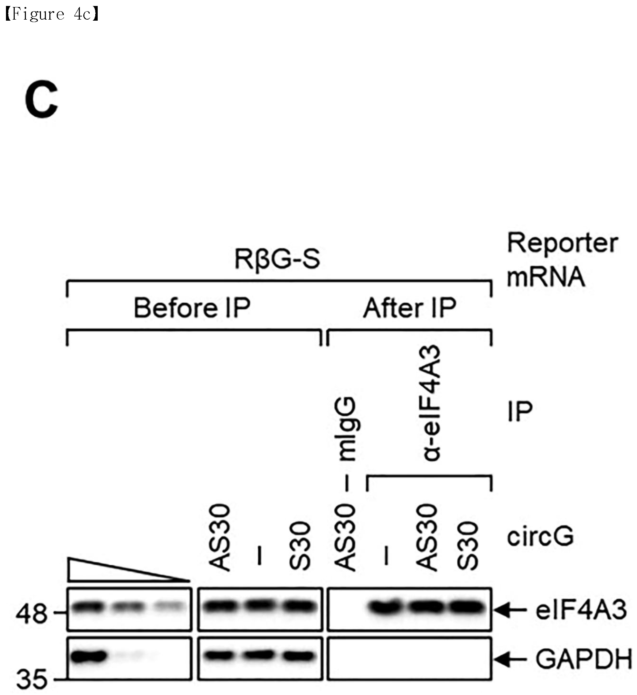

The experimental results showed that eIF4A3, one of the major components of the exon junction complex (EJC), binds to circular RNA, and that the circular RNA binds to linear reporter mRNA, as shown in Figures 4c to 4e.

### Example 4. Nonsense-mediated mRNA decay (NMD) phenomenon of linear reporter mRNA induced by circular effector RNA

We sought to confirm whether the reduction in linear reporter mRNA caused by circular effector RNA binding to the 3' untranslated region (UTR) of linear reporter mRNA was due to nonsense-mediated mRNA decay (NMD).

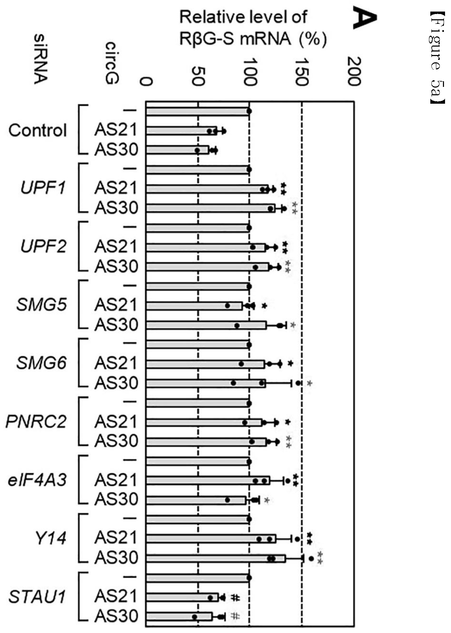

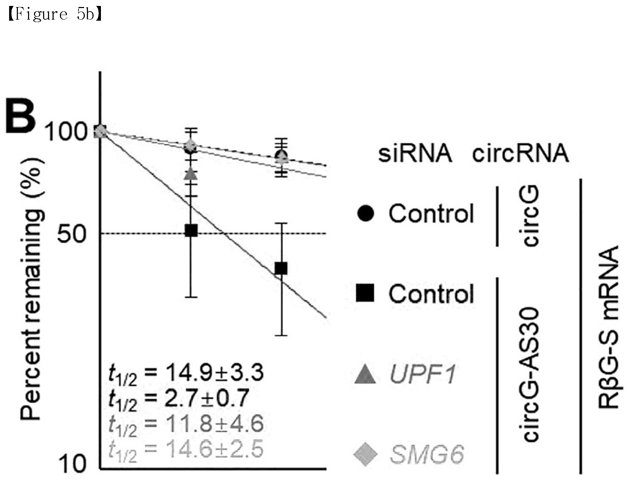

Specifically, as shown in Figures 5a, 5b, and 5c, we reduced the protein levels of NMD factors using siRNA and then observed whether the circular effector RNA caused a reduction in the amount of linear reporter mRNA.

The experimental results showed that, as depicted in Figure 5a, when the NMD factors were reduced, the decrease in linear reporter mRNA levels induced by the circular effector RNA was not observed. Additionally, as shown in Figure 5b, the stability of the linear reporter mRNA was found to increase. This indicates that the degradation of linear reporter mRNA induced by linear effector RNA is mediated by nonsense-mediated mRNA decay (NMD). Furthermore, the phenomenon where circular RNA with an exon junction complex (EJC) binds to the 3' untranslated region (UTR) of linear mRNA and induces nonsense-mediated mRNA decay (NMD) was named "nonsense-mediated mRNA decay induced by circular RNA" (circNMD). (Nonsense-mediated mRNA decay; NMD), or circNMD.

### Example 5. Identification of target mRNA and circular RNA in circNMD using microarray and RIC-seq

Microarray is an experimental method that uses fluorescent dyes to determine the types and quantities of mRNA present within cells. RIC-seq is an experimental method that identifies RNA molecules bound to RNA-binding proteins. By comprehensively analyzing the data obtained from these two experiments, we identified mRNA and circular RNA molecules that are bound to each other within cells.

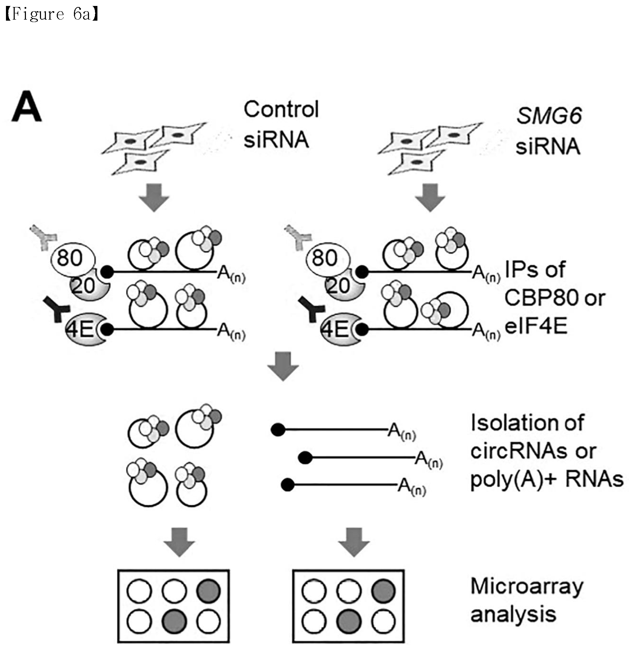

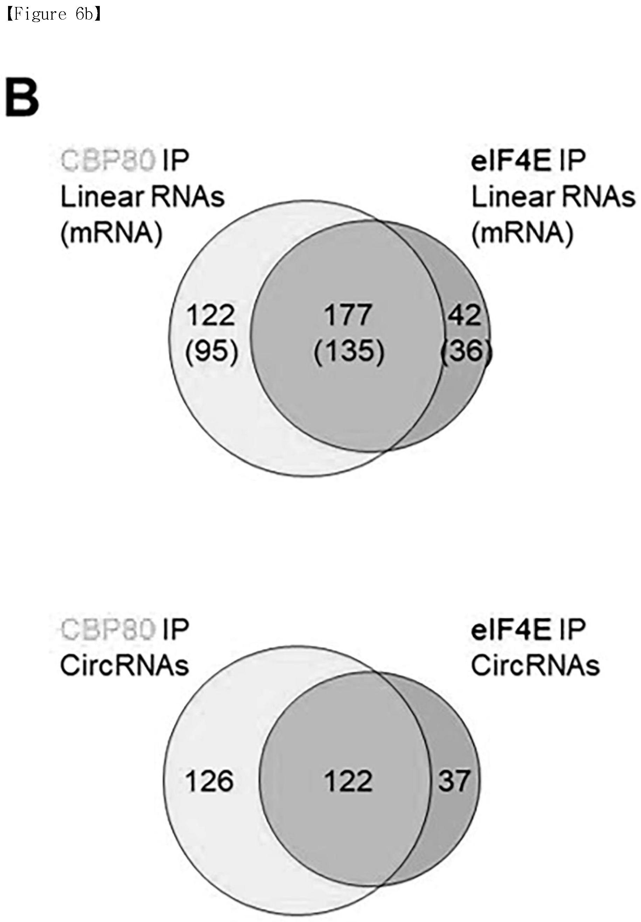

As shown in Figures 6a and 6b, when immunoprecipitation was performed using cap-binding protein (cap binding protein 80; CBP80) and the eukaryotic translation initiation factor 4E (eIF4E) were identified using immunoprecipitation, and the microarray was used to confirm which mRNAs and circular RNAs were bound.

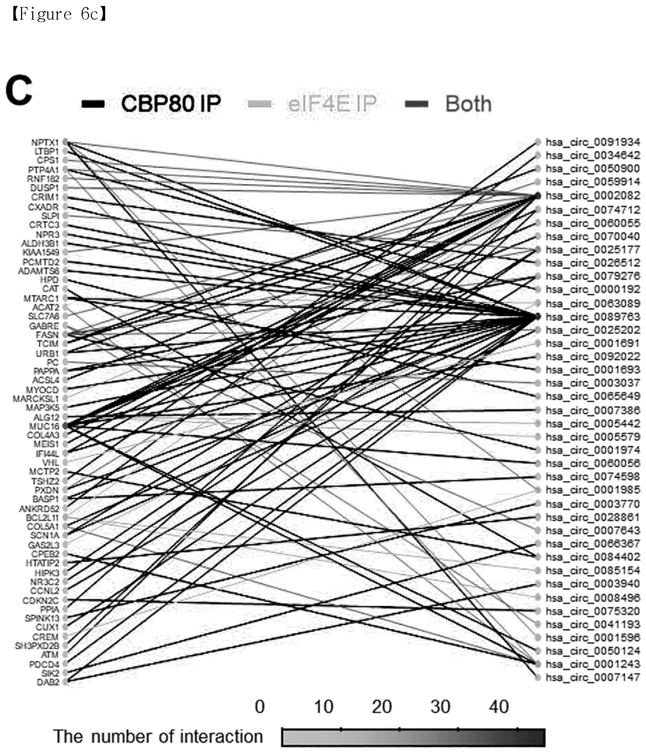

Additionally, as shown in Figure 6c, to investigate the effects of CircNMD, we analyzed only the increased mRNA when the amount of SMG6 was reduced. Furthermore, through RIC-seq data analysis, which identifies RNA-RNA interactions, we analyzed circular RNA molecules bound to mRNA. This allowed us to determine which mRNA molecules are bound to which circular RNA molecules.

### Example 6. Elucidation of the intracellular function of the CircNMD mechanism

### Example 6-1. Effects of circular RNA reduction on cells - Cell death increases when circular RNA is reduced

As shown in Figure 6 identified in Example 5, the circular RNAs hsa_circ_0002082 and hsa_circ_0008496 were found to bind to the 3' untranslated region (UTR) of BCL2L11 mRNA, thereby inducing circNMD of BCL2L11 mRNA. Furthermore, the BCL2L11 protein plays a role in increasing cell death.

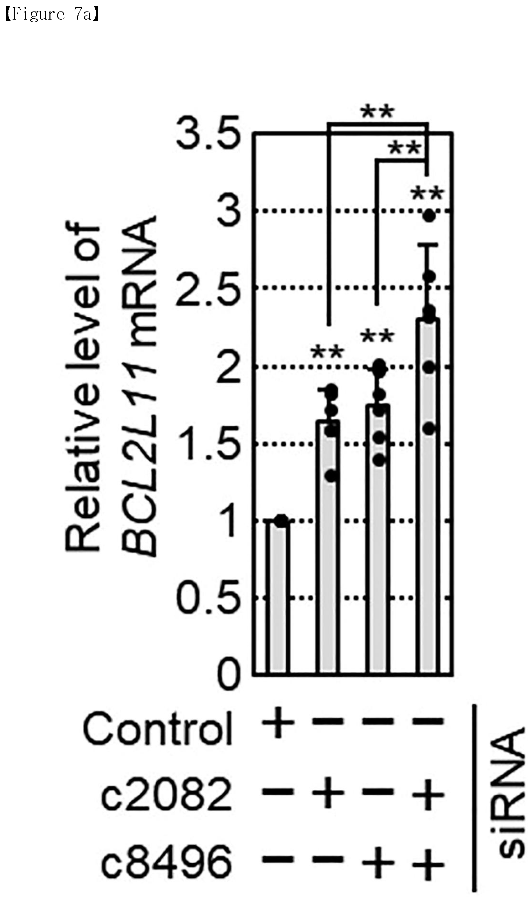

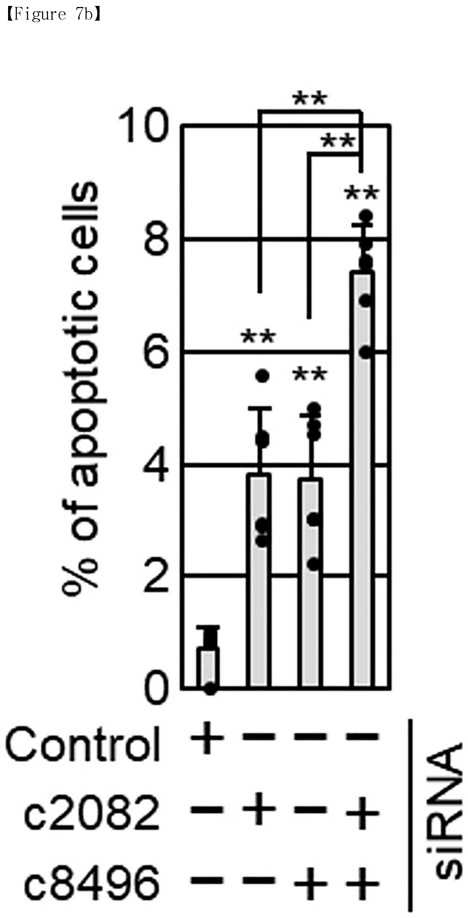

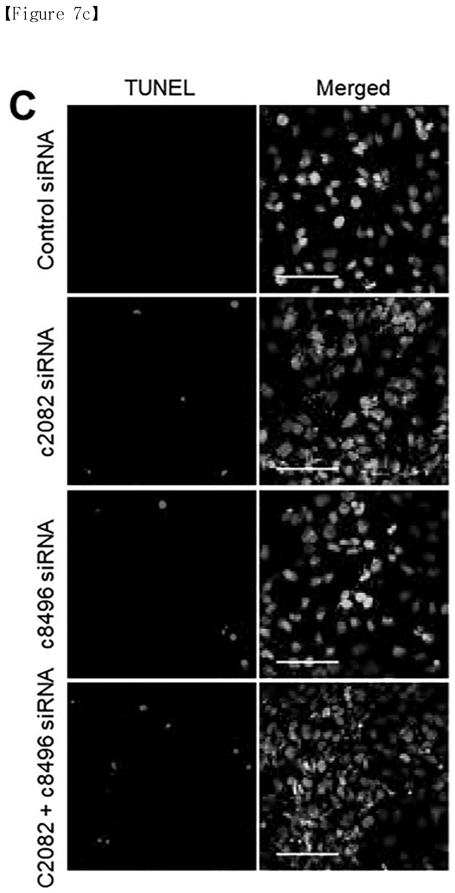

Therefore, as shown in Figure 7, we aimed to reduce the levels of hsa_circ_0002082 and hsa_circ_0008496 present in cells by using circular RNA-specific siRNA to decrease the levels of each circRNA, and then observe changes in BCL2L11 mRNA levels. Subsequently, we sought to assess the extent of cell death by conducting a TUNEL analysis (Terminal deoxynucleotidyl transferase dUTP nick end labeling assay; TUNEL assay) to assess the extent of intracellular cell death.

The experimental results showed that, as depicted in Figures 7a to 7c, when the levels of each circular RNA were reduced, the levels of the target BCL2L11 mRNA increased, and accordingly, an increase in cell death was confirmed.

### Example 6-2. Effects of circular RNA binding on cells - Inhibiting the binding of circular RNA to target mRNA increases cell death

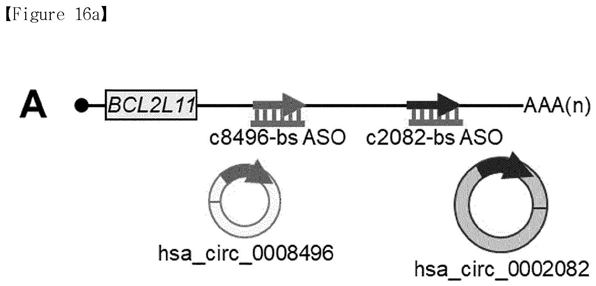

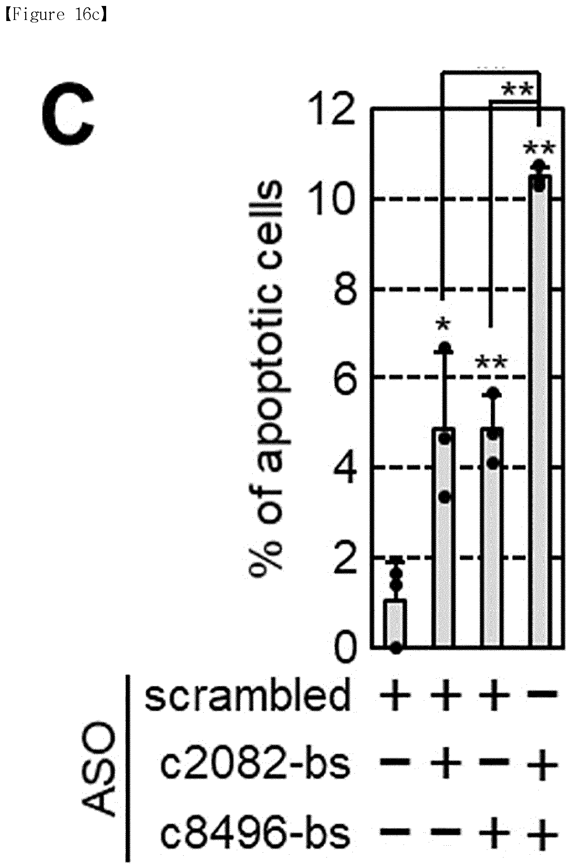

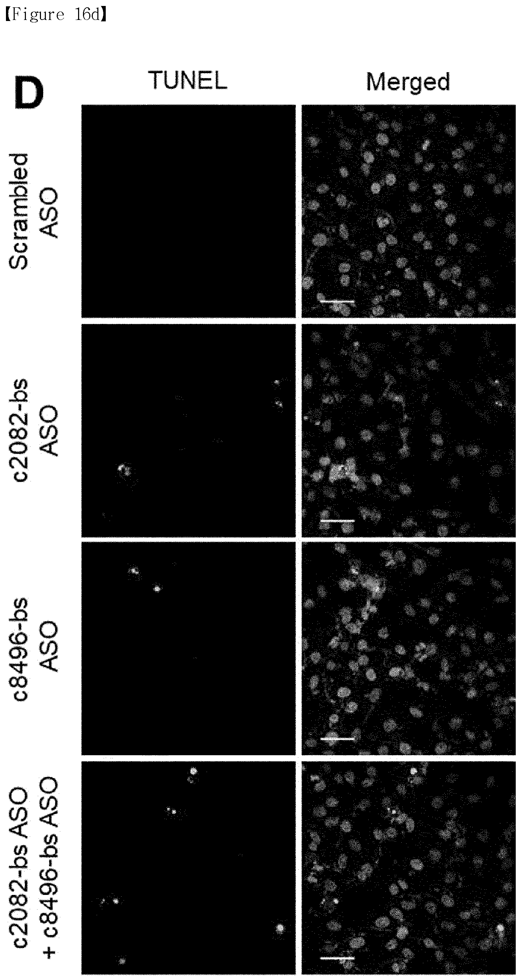

As shown in Figure 16a, we observed how cell death changes when ASOs that inhibit the binding of BCL2L11 mRNA and hsa_circ_0008496, and ASOs that inhibit the binding of BCL2L11 mRNA and hsa_circ_0002082, were treated separately and together.

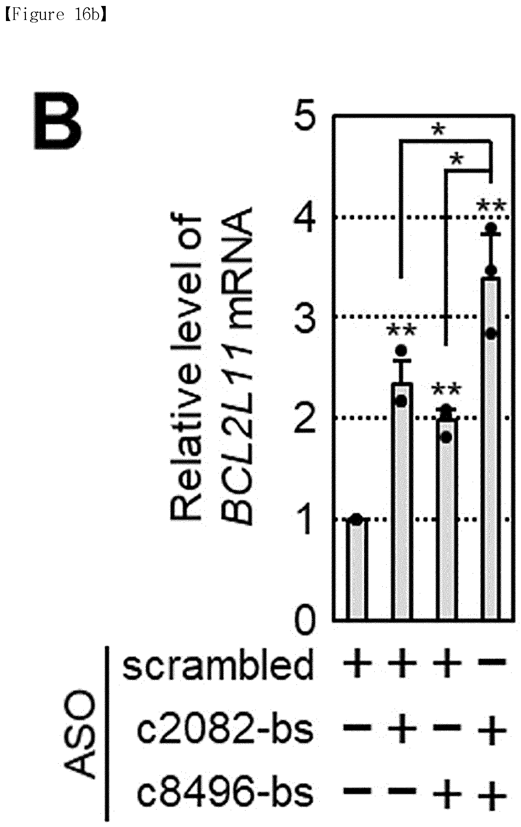

As shown in Figures 16b to 16d, when ASOs specific to the binding sites of each circular RNA were administered, an increase in BCL2L11 mRNA levels was observed, and accordingly, an increase in cell death was confirmed.

### Example 6-3. Effects of circular RNA overexpression on cells - Circular RNA overexpression reduces cell death

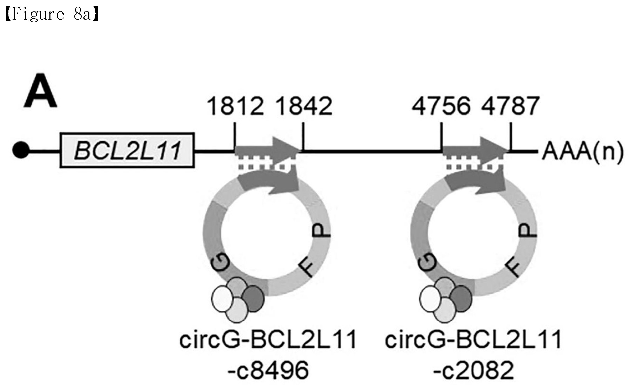

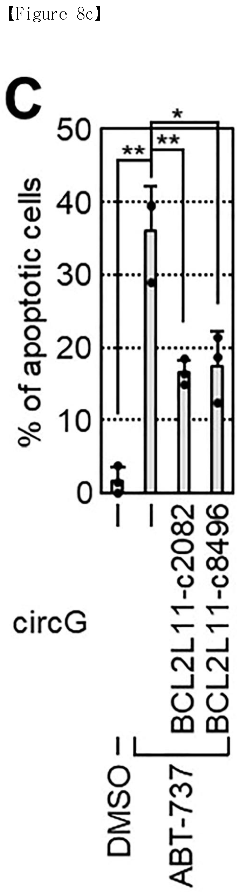

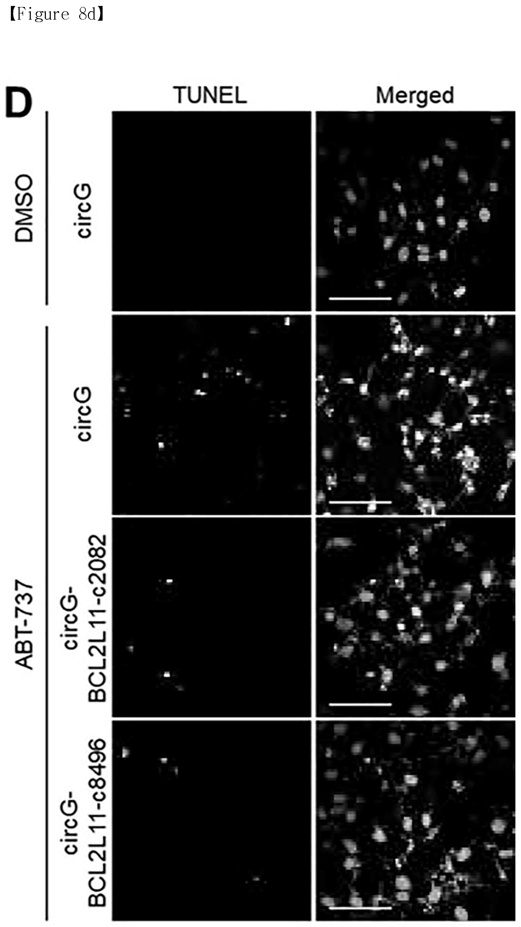

To investigate the difference in the extent of cell death when the amount of circular RNA is increased, as shown in Figure 8a, two types of circular RNA capable of binding to BCL2L11 mRNA were introduced into cells. Two types of circular RNA, circG-BCL2L11-c8496 and circG-BCL2L11-c2082, were produced to bind to endogenous BCL2L11 mRNA. Subsequently, the cells were treated with ABT-737, a drug that induces cell death, and the effects of the introduced circular RNA were observed.

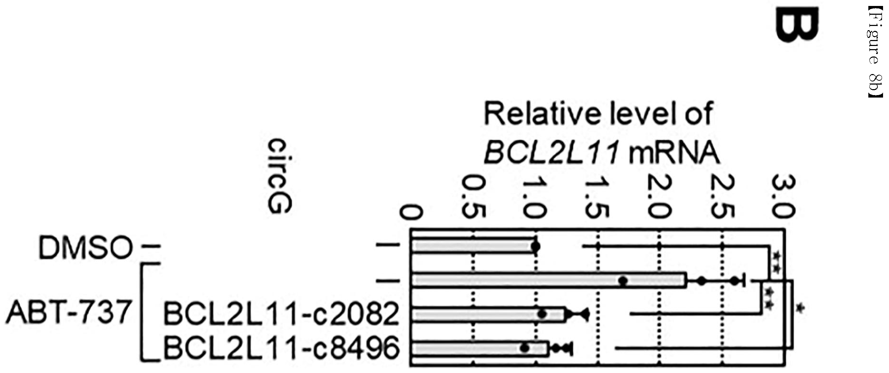

The experimental results showed that, as depicted in Figures 8b to 8d, the increased levels of BCL2L11 mRNA and cell death induced by ABT-737 treatment were reduced when circular RNA capable of binding to BCL2L11 mRNA was introduced.

### Example 6-4. Effect of artificially introduced circular RNA on circNMD - reduction in mRNA levels of a specific gene

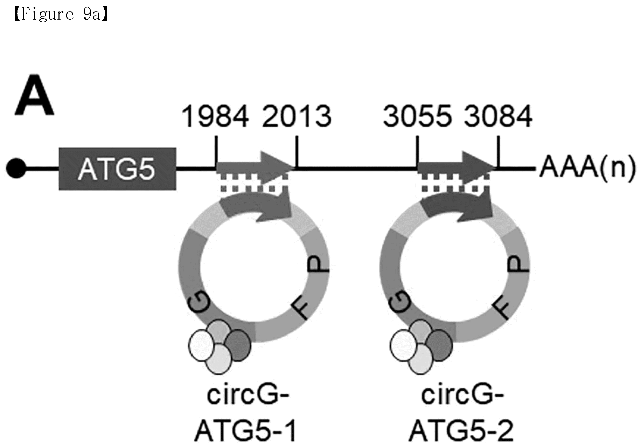

We aimed to confirm whether the amount of target mRNA could be selectively reduced through the circNMD mechanism by synthesizing circular RNA that binds to mRNA present in cells in vitro and artificially introducing it. For this purpose, since ATG5 is a protein that increases autophagy, we sought to confirm whether the amount of target mRNA could be reduced by artificially introducing circular effector RNA that binds to mRNA present in cells. As shown in Figure 9a, we synthesized two types of circular RNA that can bind to the 3'UTR of ATG5 mRNA.

Additionally, we experimentally verified whether introducing circular effector RNA into cells reduces ATG5 mRNA levels and the subsequent decrease in autophagy. We also investigated whether the effect is further enhanced when various circular effector RNA molecules are introduced.

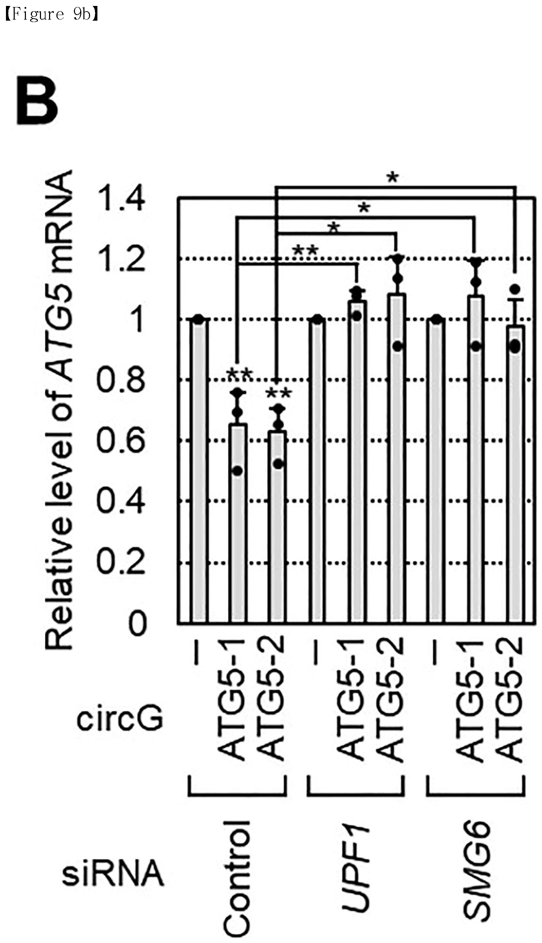

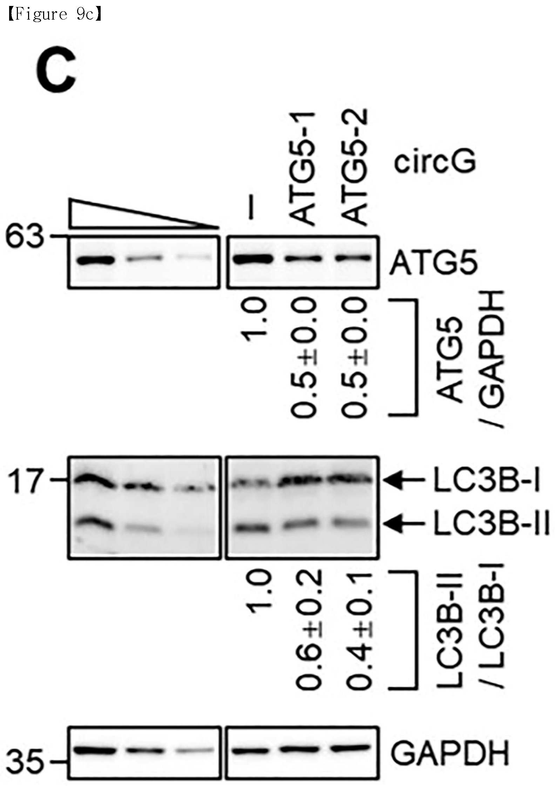

As shown in Figures 9b and 9c, the results demonstrated that artificially expressing the circular effector RNA can reduce the amount of specific mRNA, thereby decreasing the amount of the corresponding protein and regulating the cellular function of autophagy.

As described above, specific aspects of the present invention have been detailed. However, to those skilled in the art, such specific descriptions are merely preferred embodiments and do not limit the scope of the invention. Therefore, the scope of the present invention is defined by the appended claims and their equivalents.

## Claims

1. A circular nucleotide synthesized *in vitro* or generated through back-splicing *in vivo* to complementarily bind to an untranslated region (UTR) of a target mRNA in order to remove the target mRNA.

2. The nucleotide of claim 1, wherein the nucleotide is RNA or DNA encoding the same.

3. The nucleotide of claim 2, wherein the nucleotide complementarily binds to a 3'-untranslated region (3'-UTR) of the target mRNA.

4. The nucleotide of claim 3, wherein the nucleotide complementarily binds to the target mRNA at a position spaced apart by 100 or more nucleotides from a stop codon of the target mRNA.

5. The nucleotide of claim 4, wherein the circular nucleotide binds to exon junction complex (EJC) factors.

6. The nucleotide of claim 5, wherein the exon junction complex factors are at least one selected from the group consisting of eIF4A3, MNL51, Y14, and MAGOH.

7. The nucleotide of claim 6, wherein the circular nucleotide induces circNMD.

8. The nucleotide of claim 7, wherein the circNMD is mediated by nonsense-mediated mRNA decay (NMD).

9. The nucleotide of claim 8, wherein the circNMD is induced by linking the circular nucleotide and the target mRNA through an interaction between RNA-binding proteins respectively bound to the circular nucleotide and the target mRNA.

10. A composition for removing a target nucleotide, comprising the circular nucleotide of any one of claims 1 to 9.

11. The composition of claim 10, wherein the nucleotide is RNA or DNA encoding the same.

12. A method for removing a target nucleotide, comprising utilizing the circular nucleotide of any one of claims 1 to 9.

13. The method of claim 12, wherein the nucleotide is RNA or DNA encoding the same.

14. A pharmaceutical composition for preventing or treating an apoptosis-related disease, comprising the circular nucleotide of any one of claims 1 to 9 as an active ingredient.

15. The pharmaceutical composition of claim 14, wherein the apoptosis-related disease is an autoimmune disease, a cranial nervous system disease, graft-versus-host disease, organ transplant rejection, asthma, atopy, or an acute or chronic inflammatory disease.

16. The pharmaceutical composition of claim 15, wherein the cranial nervous system disease is a neurodegenerative disease or a neuroinflammatory disease.

17. The pharmaceutical composition of claim 16, wherein the neurodegenerative disease or neuroinflammatory disease is at least one selected from the group consisting of stroke, dementia, Alzheimer's disease, Parkinson's disease, Huntington's disease, Niemann-Pick disease, prion disease, Creutzfeldt-Jakob disease, frontotemporal dementia, Lewy body dementia, amyotrophic lateral sclerosis, paraneoplastic syndrome, corticobasal degeneration, multiple system atrophy, progressive supranuclear palsy, autoimmune disease of the nervous system, spinocerebellar ataxia, inflammatory and neuropathic pain, cerebrovascular disease, spinal cord injury, and tauopathy.

18. A pharmaceutical composition for preventing or treating an autophagy-mediated disease, comprising the circular nucleotide of any one of claims 1 to 9 as an active ingredient.

19. The pharmaceutical composition of claim 18, wherein the autophagy-mediated disease is at least one selected from the group consisting of cancer, a cranial nervous system disease, diabetes, a cardiomyocyte death disease, obesity, and viral infection.

20. The pharmaceutical composition of claim 19, wherein the cancer is gastric cancer, liver cancer, glioblastoma, ovarian cancer, colorectal cancer, head and neck cancer, bladder cancer, renal cell carcinoma, breast cancer, metastatic cancer, prostate cancer, pancreatic cancer, melanoma, or lung cancer.

21. A method for preventing or treating an apoptosis-related disease, comprising administering the circular nucleotide of any one of claims 1 to 9 to a subject.

22. The method of claim 21, wherein the apoptosis-related disease is an autoimmune disease, a cranial nervous system disease, graft-versus-host disease, organ transplant rejection, asthma, atopy, or an acute or chronic inflammatory disease.

23. The method of claim 22, wherein the cranial nervous system disease is a neurodegenerative disease or a neuroinflammatory disease.

24. The method of claim 23, wherein the neurodegenerative disease or neuroinflammatory disease is at least one selected from the group consisting of stroke, dementia, Alzheimer's disease, Parkinson's disease, Huntington's disease, Niemann-Pick disease, prion disease, Creutzfeldt-Jakob disease, frontotemporal dementia, Lewy body dementia, amyotrophic lateral sclerosis, paraneoplastic syndrome, corticobasal degeneration, multiple system atrophy, progressive supranuclear palsy, autoimmune disease of the nervous system, spinocerebellar ataxia, inflammatory and neuropathic pain, cerebrovascular disease, spinal cord injury, and tauopathy.

25. A method for preventing or treating an autophagy-mediated disease, comprising administering the circular nucleotide of any one of claims 1 to 9 to a subject.

26. The method of claim 25, wherein the autophagy-mediated disease is at least one selected from the group consisting of cancer, a cranial nervous system disease, diabetes, a cardiomyocyte death disease, obesity, and viral infection.

27. The method of claim 26, wherein the cancer is gastric cancer, liver cancer, glioblastoma, ovarian cancer, colorectal cancer, head and neck cancer, bladder cancer, renal cell carcinoma, breast cancer, metastatic cancer, prostate cancer, pancreatic cancer, melanoma, or lung cancer.
